# FASCICULE DE BREVET EUROPEEN

(11) **EP 4 121 133 B1**
(45) Date de publication et mention de la délivrance du brevet: **16.08.2023**
(21) Numéro de dépôt: 21711292.9
(22) Date de dépôt: 17.03.2021
(51) Int. Cl.: A61L 27/16, A61L 27/18, A61L 27/52

(54) **HYDROGEL HÉTÉROGÈNE HYBRIDE, PROCÉDÉ DE FABRICATION ET UTILISATION COMME IMPLANT DE COMBLEMENT NON-DÉGRADABLE IN-SITU**
HYBRIDES HETEROGENES HYDROGEL, HERSTELLUNGSVERFAHREN UND VERWENDUNG ALS NICHT ABBAUBARES IN-SITU-FÜLLERIMPLANTAT
HYBRID HETEROGENEOUS HYDROGEL, MANUFACTURING METHOD AND USE AS AN IN-SITU NON-DEGRADABLE FILLLER IMPLANT

(30) Priorité: 17.03.2020 FR 2002619
(43) Date de publication de la demande: 25.01.2023
(73) Titulaire: NEUROBIOMAT, 74160 Archamps (FR)
(72) Inventeur: WOERLY, Stéphane, 74160 Saint Julien en Genevois (FR)
(74) Mandataire: Talbot, Alexandre
(86) Numéro de dépôt international: PCT/EP2021/056753
(87) Numéro de publication internationale: WO 2021/185881

(56) Documents cités:
- WO-A1-98/16266
- WO-A1-2010/097524

## Description

### Domaine technique

L'invention concerne un hydrogel hétérogène hybride et plus particulièrement son utilisation en tant qu'implant de comblement.

### Technique antérieure

Une lésion du parenchyme nerveux de la moelle épinière qu'elle soit consécutive à un traumatisme focal, une ischémie, une chirurgie d'exérèse d'une tumeur ou d'une malformation vasculaire ou d'autres causes entraine une rupture des connexions des fibres nerveuses ce qui interrompt la transmission de l'influx nerveux de la commande des fonctions motrices depuis le cerveau et en retour le traitement des fonctions sensitives. Il en résulte une paralysie complète ou partielle.

Dans le cas d'un traumatisme de la moelle épinière, la lésion par compression ou par tout autre impact vertébro-médullaire évolue par des phénomènes neurodégénératifs qui se développent de façon centrifuge à partir de l'épicentre de la lésion et qui conduisent progressivement vers le stade chronique de la lésion initiale. Au stade chronique, la lésion est représentée par une cavité kystique, cloisonnée et circonscrite par un tissu cicatriciel hétérogène composé de cellules gliales, de fibroblastes, de péricytes et de cellules méningées, de molécules de la matrice extracellulaire notamment des protéoglycanes et du collagène. Cette forme de cicatrisation, tout comme la cavité intra médullaire, est cause d'échec à la régénération des fibres nerveuses des voies axonales ascendantes et descendantes.

Les thérapies régénératrices visent la réparation cellulaire de la cavité kystique au stade chronique du traumatisme médullaire ainsi que la revascularisation de la zone endommagée qui peut représenter une perte de volume importante dont la taille varie de 2 à 6 corps vertébraux de hauteur. Les thérapies régénératrices visent également à faire recroître les fibres nerveuses à travers cette cavité kystique dans le but de favoriser la reconnexion des circuits neuronaux spinaux, et rétablir les fonctions locomotrices et sensitives. Cette stratégie prend en compte la plasticité post lésionnelle du système nerveux qui est dans la capacité de reformer des circuits relais entre les fibres nerveuses en repousse et des neurones intacts sous lésionnels. La stratégie utilisée en ingénierie tissulaire est d'introduire un substrat poreux et permissif au niveau de la lésion (soit au stade aigue ou au stade chronique) pour servir de support physique, chimique et mécanique aux processus endogènes de réparation cellulaire, vasculaire et nerveux conduisant à la reconstruction histologique du tissu nerveux.

Les supports généralement utilisés au niveau expérimental et dans des essais cliniques sont représentés par la classe particulière des biomatériaux, les hydrogels. Ce sont des matrices de polymères formant un réseau macromoléculaire réticulé saturé en eau. Ils sont utilisés en ingénierie tissulaire, notamment au niveau du système nerveux. Ils sont préparés à partir de polymères dégradables ou biorésorbables pour constituer des structures poreuses temporellement instables. Ces polymères sont d'origine naturelle : alginate, agarose, chitosan, collagène, acide hyaluronique, fibrine, peptides ou de synthèse comme les poly caprolactones, les poly(hydroxybutyrates), les poly(ortho esters), les poly(a-hydroxy esters), les polyanhydrides. Ainsi des hydrogels dégradables et/ou bioresorbables ont été proposés pour favoriser la régénération nerveuse des lésions de la moelle épinière sur des modèles expérimentaux ou en essais cliniques chez l'homme.

Les hydrogels biodégradables sont ceux qui, une fois introduits dans un organisme vivant, se décomposent par hydrolyse chimique spontanée pour les polyanhydrides, les poly(ortho esters), les poly(a-hydroxy esters). Les thioether-esters se décomposent en présence d'eau et les polymères biologiques (oligopepetides, protéines, polysaccharides) se décomposent par l'action d'enzymes ou d'autres protéines produites par les cellules.

Par exemple le brevet US 7,163,545 divulgue une matrice d'acide poly(lactique-co-glycolique), incluant des canaux-guides pour la régénération axonale en combinaison avec des agents thérapeutiques. Le brevet US 8,377,463 enseigne un dispositif formé d'acide poly(lactique-co-glycolique) pour traiter le stade aigue des lésions de la moelle épinière qui se dégrade in situ entre 30 et 60 jours, pouvant être combiné avec des agents thérapeutiques et/ou des cellules souches. Le document US 2018/0037865 décrit un hydrogel complexe comprenant une matrice dégradable d'acide hyaluronique, de collagène, de fibrines, de chitosane, de methycellulose, de poly (oxyde d'éthylène) ou une combinaison entre eux, combinée à des cellules souches et des molécules thérapeutiques, qui se dégradent in situ à une vitesse variable selon la nature du polymère. Le document US 20060002978 divulgue une matrice tubulaire contenant un matériau polymérique poreux formé d'homo- ou de copolymères d'acide lactique et/ou d'acide glycolique et/ou de poly (caprolactone). La matrice polymère peut comprendre un polyester aliphatique, un polyanhydride, un polyphosphazène, un alcool polyvinylique, un polypeptide, un alginate. Le brevet US 8,877,498 enseigne une matrice comportant une structure hiérarchisée avec des canaux de topographie alignés et des arêtes le long des parois permettant une régénération guidée des fibres nerveuses, et dont la composition comprend du chitosan, de la chitine, de la cellulose, de l'alginate, de la gélatine, de l'acide hyaluronique, du collagène, de l'élastine ou une de leur combinaison. Le document WO2014013188 divulgue un biomatériau de chitosane acétylé sous la forme d'une suspension de microgels ou sous la forme de gel de 2 à 3 mm³ pour traiter les lésions de la moelle épinière. Les documents US 2015/0166786 A1 et WO2013010087A1 révèlent un hydrogel composé de poly(lactique-co-glycolique) ou comprenant du poly(ε-caprolactone) combiné à de la poly(L-lysine) pour traiter les lésions aigues de la moelle épinière chez l'homme. Le document US 2015/0044259 A1 décrit une matrice composée de poly-D Lysine et d'un peptidoglycane pour favoriser la croissance de fibres nerveuses. Le document EP2347763A1 et le document US 2011/0177170 A1 décrivent une matrice composée de microparticules de collagène incluses dans un gel homogène composé de peptides, d'acide uronique et d'hexosamine pour implantation conjointement avec une greffe cellulaire dans le traitement de lésions du système nerveux central, et dont sa dégradation s'opère entre quelques semaines et plusieurs mois. Le document WO2013/084137 divulgue un implant dégradable à partir de sulfate de calcium hémi hydraté qui inclut des canaux de géométrie parallèle combiné à un facteur de croissance pour le traitement de lésions complètes de la moelle épinière chez l'homme.

Il est aussi proposé des hydrogels biodégradables tels que ceux présentés dans le document US 8,815,277 ou dans le document WO2011/002249. Il est indiqué que ces systèmes présentent l'avantage de ne pas être invasifs, c'est-à-dire qu'ils ne nécessitent pas une chirurgie à ciel ouvert de la moelle épinière pour leur implantation. En général, ces systèmes se dégradent in situ en deux semaines, ce qui est très rapide par rapport à un processus régénératif.

Il est également proposé un hydrogel à base d'acide poly (lactique-co-glycolique) pour réparer les voies nerveuses de la moelle épinière. Cet hydrogel se dégrade en 30 à 60 jours chez le rat, ce qui est une durée très insuffisante pour une régénération tissulaire aboutie si on transpose ces résultats à l'homme. Une étude comparative a montré que le délai de croissance des axones isolés est plus long et la vitesse de régénération est trois fois plus lente chez l'humain comparé au rat (Gordon 2007, The potential of electrical stimulation to promote functional recovery after peripheral nerve injury-comparisons between rats and humans ; Acta Neurochir Suppl. 2007; 100:3-11). Dans le cas de la moelle épinière, la durée est encore supérieure car un grand nombre de fibres nerveuses doivent repousser pour assurer une récupération fonctionnelle motrice. La croissance des axones à travers le site de réparation chirurgicale est lente et asynchronisée jusqu'à atteindre les cibles appropriées du segment sous lésionnel de la moelle épinière.

Ces méthodes pour tenter de réparer des lésions de la moelle épinière ont une efficacité très limitée et imparfaite dans la régénération du tissu nerveux central dans une application à l'homme car elles ne permettent pas une réparation des structures anatomiques du segment médullaire endommagé concomitamment à une récupération fonctionnelle des fonctions neurologiques.

Une fois implantées dans l'organe, les matrices hydrogels dégradables se dégradent par rupture des chaines de polymères simultanément avec la migration et la colonisation des cellules, des vaisseaux sanguins, des fibres nerveuses en repousse au cours du remodelage tissulaire. Ainsi, lors de la reconstruction tissulaire de la moelle épinière, pour faciliter la formation de néotissu nerveux, les matrices hydrogels dégradables présentent un fort taux de dégradation conduisant à une perte rapide des propriétés mécaniques initiales de support. Pour que la régénération tissulaire soit optimale c'est-à-dire complète dans le temps, il est essentiel que le support hydrogel conserve tout au long du processus de remodelage tissulaire une certaine intégrité structurelle spatiale et temporelle.

Il ressort également que l'hydrolyse des liaisons esters des poly (α-hydroxyacides) libère des composés acides qui en s'accumulant dans le site de greffe diminue le pH. La diminution du pH provoque une accélération de la vitesse d'hydrolyse dans le centre de l'implant comparé à la surface, provoquant une perte rapide des propriétés mécaniques initiales de l'implant et une réaction locale à corps étranger comme illustré dans Bostman OM and Pihlajamaki HK (2000) Adverse tissue reactions to bioabsorbable fixation devices. Clin Orthop Rel Res 371 : 216-227*.*

Le document WO2010/097524 divulgue un hydrogel hétérogène hybride qui est un copolymère dérivé des monomères suivants :
- un monomère dendrimère comportant un noyau central A, des branches dendritiques macromoléculaires de poly(oxyéthylène) et au moins une des branches dendritiques fonctionnalisée par un radical acrylate ou méthacrylate,
- un méthacrylamide N-substitué ou acrylamide N-substitué, et
- un matériau copolymérisable bioactif choisi parmi le groupe consistant en un dérivé d'un sucre complexe, un dérivé d'un peptide d'adhérence à un tissu et un dérivé d'un conjugué polymère couplé à des anticorps dirigés contre des dérivés lipidiques

### Objet de l'invention

Un objet de l'invention consiste à pallier ces inconvénients, et plus particulièrement à fournir un implant à base d'un hydrogel dont la vitesse de dégradation est réduite en comparaison des hydrogels de l'art antérieur et qui s'adaptent mieux aux contraintes mécaniques liées à la colonisation cellulaire.

La portée de l'invention est définie par les revendications. Les modes de réalisation relatifs à des méthodes de traitement ne sont pas couverts par l'objet des revendications.

Selon un aspect de l'invention, il est proposé un hydrogel hétérogène hybride selon les revendications 1 à 11 formé au moyen d'un copolymère dérivé d'au moins trois des monomères suivants :
- un monomère dendrimère fonctionnalisé par une seule branche munie d'un radical éthylénique insaturé,
- un composé acrylamide choisi parmi un méthacrylamide N-substitué et un acrylamide N-substitué, et
- un agent de réticulation.

L'hydrogel hétérogène hybride est caractérisé en ce qu'il est formé majoritairement par une pluralité de microbilles ayant un diamètre supérieur à 1,5 microns et inférieur à 10 microns et contenant majoritairement en poids le méthacrylamide N-substitué et acrylamide N-substitué, les microbilles étant assemblées les unes aux autres pour former des agrégats contenant entre 5 et 50 microbilles, les agrégats étant liés les uns aux autres par des points de réticulation pour définir un réseau poreux traversant définissant des chemins percolants tridimensionnels, le réseau poreux traversant définissant des pores dont la majorité de la fraction poreuse est formée par des pores dont le diamètre est compris entre 10 et 30 microns et dans lequel l'hydrogel hétérogène hybride a un caractère viscoélastique et possède un module élastique compris entre 1 et 200kPa.

Selon un développement, les microbilles ont un diamètre compris entre 2 et 5 microns.

Préférentiellement, les microbilles comportent au moins 90% en poids du composé acrylamide. Encore plus préférentiellement, les microbilles sont constituées du composé acrylamide réticulé.

Dans un mode de réalisation particulier, les agrégats contiennent entre 10 et 30 microbilles. Il est avantageux de prévoir que les agrégats soient déformables en compression.

Avantageusement, le monomère dendrimère fonctionnalisé comporte une ou plusieurs branches dendritiques macromoléculaire de poly(oxyde d'éthylène), ladite au moins une branches dendritiques macromoléculaire de poly(oxyde d'éthylène) étant fonctionnalisée par un ou plusieurs matériaux copolymérisables bioactifs choisis parmi le groupe consistant en un dérivé d'un sucre complexe, un dérivé d'un peptide d'adhérence à un tissu et un dérivé d'un conjugué polymère couplé à des anticorps dirigés contre des dérivés lipidiques lesdits un ou plusieurs matériaux copolymérisables bioactifs recouvrant la paroi du réseau percolant tridimensionnel.

De manière préférentielle, la paroi du réseau percolant tridimensionnel est fonctionnalisée au moyen de plusieurs matériaux copolymérisables bioactifs différents.

Dans une configuration avantageuse, lesdites une ou plusieurs molécules actives sont choisies parmi le groupe consistant en un dérivé de sucres complexes, de dérivés de peptide d'adhérence à un tissu ou de peptide avec une activité angiogénique, de dérivés de peptide de stimulation de la repousse nerveuse, de dérivés de peptide de stimulation de la prolifération et la différenciation cellulaire, un dérivé d'un conjugué polymère couplé à des anticorps dirigés contre des dérivés lipidiques, des chimiokines de la classe des Stromal-derived factor-1 (SDF-1).

Il est encore intéressant de prévoir que le monomère dendrimère fonctionnalisé comporte un noyau central A, des branches dendritiques macromoléculaire de poly(oxyde d'éthylène) avec au moins une des branches dendritiques fonctionnalisées par un radical acrylate ou méthacrylate polymérisable.

Selon une autre configuration, le composé acrylamide est le N-(2-hydroxypropyl)méthacrylamide (HPMA) et le monomère dendrimère fonctionnalisé comporte des branches dendritiques de poly(oxyde d'éthylène) fonctionnalisés à leur périphérie avec des agents bioactif conférant des propriétés bioactives pour la régénération tissulaire.

Préférentiellement, l'hydrogel hétérogène hybride comme implant de comblement est utilisé pour remplir une cavité intra-parenchymateuse du système nerveux central.

De manière avantageuse, l'hydrogel hétérogène hybride comme implant de comblement est utilisé pour corriger la malformation congénitale du système nerveux centrale ou la spina bifida.

Selon un aspect de l'invention, il est proposé un procédé de fabrication d'un hydrogel hétérogène hybride selon les revendications 12 à 14 qui soit facile à mettre en oeuvre pour la production industrielle, permettant la production d'hydrogels hétérogènes hybrides mieux adaptés à la formation d'un implant de comblement et notamment en ce qui concerne les spécifications physico-chimiques.

Le procédé de fabrication d'un hydrogel hétérogène hybride est remarquable en ce qu'il comporte :
- la formation de microbilles par séparation de phase induite par copolymérisation et copolymérisation radicalaire libre à une température comprise entre 45°C et 55°C à partir d'un mélange réactionnel comportant au moins trois des monomères suivants :
   - un monomère dendrimère fonctionnalisé par une seule branche comportant un radical éthylénique insaturé, les autres branches étant dépourvues de radical éthylénique,
   - un composé acrylamide choisi parmi un méthacrylamide N-substitué et acrylamide N-substitué, et
   - au moins un agent de réticulation éthylénique insaturé bifonctionnel comportant deux liaisons vinyliques réactives, et
   un initiateur à radicaux libres,
   les microbilles ayant un diamètre supérieur à 1,5 microns et inférieur à 10 microns et contenant majoritairement en poids le méthacrylamide N-substitué et l'acrylamide N-substitué,
- les microbilles étant assemblées les unes aux autres pour former des agrégats contenant entre 5 et 50 microbilles, les agrégats étant liés les uns aux autres par des points de réticulation pour définir un hydrogel hétérogène hybride délimitant un réseau poreux traversant définissant des chemins percolants tridimensionnels, le réseau poreux traversant définissant des pores dont la majorité de la fraction poreuse est formée par des pores dont le diamètre est compris entre 10 et 30 microns et
   dans lequel l'hydrogel hétérogène hybride a un caractère viscoélastique et possède un module élastique compris entre 1 et 200kPa ; et
   dans lequel le mélange réactionnel est injecté dans des moules de forme cylindrique étanche et conducteur de la chaleur

Dans un développement, le rapport molaire entre le monomère dendrimère fonctionnalisé et l'agent de réticulation est compris entre 0,1 et 0,8. Préférentiellement, le monomère dendrimère fonctionnalisé possède une masse moléculaire est comprise entre 6220g/mol et 23280g/mol.

Préférentiellement, un matériau copolymérisable bioactif est présent dans le mélange réactionnel pour former les microbilles, le matériau copolymérisable bioactif étant choisi parmi le groupe consistant en un dérivé d'un sucre complexe, un dérivé d'un peptide d'adhérence à un tissu et un dérivé d'un conjugué polymère couplé à des anticorps dirigés contre des dérivés lipidiques.

Dans une configuration avantageuse, le mélange réactionnel est injecté dans un moule en métal dont les parois internes sont recouvertes de polytétrafluoroéthylène. Le moule est préférentiellement chauffé au moyen d'un bain marie. Préférentiellement, la polymérisation du mélange réactionnel est réalisée à une première température pendant au moins 80 minutes pour former les microbilles puis une augmentation de la température du moule et du mélange réactionnel d'au moins 5°C est effectuée.

De manière avantageuse, le procédé de formation d'agrégats de microbilles d'un hydrogel hétérogène hybride selon une des configurations précédentes intervient dans un procédé de fabrication d'un implant de comblement. Le procédé de fabrication d'un implant de comblement comportant la formation d'agrégats de microbilles d'un hydrogel hétérogène hybride et l'assemblage des agrégats les uns aux autres par un procédé d'impression tridimensionnelle pour former un implant de comblement.

### Description sommaire des dessins

D'autres avantages et caractéristiques ressortiront plus clairement de la description qui va suivre de modes particuliers de réalisation et de mise en oeuvre de l'invention donnés à titre d'exemples non limitatifs et représentés aux dessins annexés, dans lesquels :
- la figure 1 illustre de manière schématique un implant muni d'un hydrogel hétérogène hybride définissant des canaux traversants tridimensionnels ;
- la figure 2 illustre un corps métallique définissant quatre puits cylindriques dont les parois sont revêtues d'une surface en PTFE.

### Description des modes de réalisation

Un implant à base d'hydrogel est destiné à être implanté dans une cavité représentant une perte volumétrique de tissu, par exemple une cavité médullaire. Si la vitesse de dégradation (V_{d}) de l'hydrogel est supérieure à la vitesse de la régénération (Vᵣ) cellulaire, rapidement, il n'y a plus de support pour la reconstruction tissulaire. La reconstruction tissulaire sera limitée à la périphérie de la zone d'implantation et le processus cellulaire de régénération sera incomplet. De plus, la biodégradation des échafaudages de polymères implantés dans la lésion médullaire entraîne la séparation physique entre le corps de l'implant et le tissu de la moelle épinière. L'espace de séparation entre l'implant et le tissu est rempli de liquide céphalo-rachidien et empêche l'intégration de l'hydrogel dans le tissu nerveux. Cela empêche également les axones en cours de régénération d'atteindre le corps de l'échafaudage formé par l'implant.

La génération de produits de dégradation acides peut entraîner une réponse inflammatoire aigue. D'autres produits de dégradation peuvent avoir des effets toxiques au niveau cellulaire et perturber l'homéostasie d'un organe. Ces produits de dégradations sont générés dans le temps jusqu'à complète destruction du support polymère. Les produits de dégradations peuvent être transportés par la circulation systémique pour s'accumuler de façon séquentielle dans des organes cibles dont les conséquences à long terme peuvent être délétères pour l'organe.

Pour que le processus de régénération soit optimal et complet, il faut que la vitesse de dégradation du réseau polymère de l'implant soit en équilibre avec la vitesse de réparation tissulaire de manière à ce que la décroissance du volume total du gel avec le volume croissant de la biocharge cellulaire en progression reste constant pour ne pas imposer des contraintes mécaniques de compression aux interfaces avec l'organe hôte. De telles contraintes provoqueraient des lésions par compression ischémique. Même s'il est possible de contrôler la vitesse de dégradation d'un hydrogel de polymères in vitro sous des conditions expérimentales strictement contrôlées, il est impossible de contrôler in vivo la vitesse de dégradation de la matrice hydrogel et de suivre in vivo la vitesse de dégradation de la matrice polymère par rapport à la vitesse de biocharge cellulaire. Il est donc impossible d'utiliser efficacement un implant biodégradable dans un processus de régénération.

En général, le processus de régénération cellulaire et le processus de dégradation de l'implant débutent depuis l'interface en contact avec la moelle épinière pour s'étendre vers l'intérieur de la cavité. De plus, le centre de la matrice en hydrogel ne se dégrade pas complètement et reste sous forme de chaines oligomères ayant perdu leur structure et leur fonction de substrat de croissance. L'implant n'est plus utilisable car il ne fournit aucun support mécanique et il finit par gêner la reconstruction.

La dégradation de la matrice peut encore entrainer une dissociation des connexions qui existent entre les cellules qui ont migré dans la structure de l'hydrogel. Ces connexions sont essentielles dans la formation et la cohésion d'un tissu fonctionnel.

L'utilisation d'un hydrogel de polymères dégradables comme implant en ingénierie tissulaire pour la réparation optimale d'une perte de volume tissulaire et notamment du système nerveux nécessite donc de tenir compte à la fois de l'évolution du comportement mécanique de la matrice hydrogel au cours de son utilisation pour que celle-ci conserve son intégrité structurelle jusqu'à la formation du nouveau tissu biologique et de sa vitesse réelle de dégradation in vivo. Ceci n'est pas possible puisque, au fur et à mesure de sa dégradation, la matrice perd progressivement sa topographie architecturale et donc ses propriétés mécaniques qui sont définies par son architecture initiale. L'intégrité structurelle de l'implant ne peut être maintenue dans le temps. Or la stabilité structurelle de l'implant est une caractéristique essentielle pour soutenir la formation de la reconstruction tissulaire durant toute la reconstruction tissulaire.

Suite à une lésion, les fibres nerveuses ont une capacité naturelle à se régénérer soit par élongation, soit par bourgeonnement collatéral. Cette capacité est fortement réduite si les extrémités des fibres en régénération, les cônes de croissance, ne trouvent pas de substrat pour adhérer et s'allonger.

Or, si la matrice se dégrade, le substrat par lequel les cônes de croissance peuvent progresser dans le processus de régénération axonale ne peut être continu. Il est donc avantageux d'utiliser un implant réalisé dans un hydrogel dont la vitesse de dégradation V_{d} est inférieure ou égale à la vitesse de régénération Vᵣ du tissu.

Il est particulièrement intéressant d'utiliser un hydrogel hétérogène hybride qui est non-dégradable, c'est-à-dire dont la vitesse de dégradation est inférieure à la vitesse de régénération. Préférentiellement, par hydrogel hétérogène hybride non-dégradable, on entend une composition polymérique qui n'est pas sujet à une dégradation in situ par réaction d'hydrolyse chimique ou enzymatique ou de clivage par photolyse, dans des conditions physiologiques représentatives du corps humain. Par exemple, la dégradation chimique est faible ou nulle sur une période de référence au moins égale à un ou deux ans.

L'étude de la dégradabilité de l'hydrogel hétérogène hybride est avantageusement réalisée à 40°C dans une solution acide dont le pH est égal à 1 et dans une solution basique dont le pH est égal à 14. Un morceau d'hydrogel est placé dans chacune de ces solutions et chaque solution est maintenue à 40°C, par exemple au moyen d'une plaque chauffante. La solution est agitée. L'échantillon d'hydrogel hétérogène hybride et la solution sont observés régulièrement. Par exemple, on constate qu'au bout d'une semaine, un hydrogel hétérogène hybride non-dégradable n'a pas changé d'aspect et la solution est restée limpide. Aucun résidu flottant de l'échantillon n'est observé dans la solution. On ne note pas non plus une perte de masse de l'hydrogel.

La solution acide est avantageusement une solution d'acide chlorhydrique à 0,1 mol/L. la solution basique est avantageusement une solution de soude à 1 mol/L.

L'hydrogel hétérogène hybride est également analysé par chromatographie en phase liquide à haute performance. Un mélange contenant 25% volumique de méthanol et 75% d'eau est préférentiellement utilisé comme éluant. Le débit de l'éluant est avantageusement de 1mL/min. La colonne utilisée peut être de type C18 novapack 3.9*150mm en phase inverse. L'analyse des chromatogrammes permet de détecter la dégradation de l'hydrogel en recherchant ses constituants.

Avec un hydrogel hétérogène hybride non dégradable, l'analyse des chromatogrammes montre très peu de nouveaux pics au cours du temps. Par exemple, les chromatogrammes ont été analysés afin de suivre les principaux constituants d'un hydrogel selon l'invention, par exemple un hydrogel contenant du HPMA (N-(2-Hydroxypropyl) methacrylamide). Les analyses de chromatographie en phase liquide à haute performance ne montrent aucune dégradation de l'hydrogel à base de HPMA. Ces observations sont corroborées avec des tests « in vivo » où le gel à base de HPMA est implanté dans une moelle épinière. L'analyse du liquide céphalo-rachidien par électrophorèse ne montre aucun produit de dégradation de type oligomère dans le liquide céphalo-rachidien. Cette absence de produit de dégradation montre une absence de dégradation de l'hydrogel.

Il est également avantageux d'avoir un hydrogel qui n'est pas bioresorbable et qui est compressible. Un tel hydrogel permet son utilisation en tant qu'implant de comblement et son interposition entre les bords d'un défaut anatomique d'un organe par exemple du système nerveux et notamment une cavité kystique intramédullaire post-traumatique

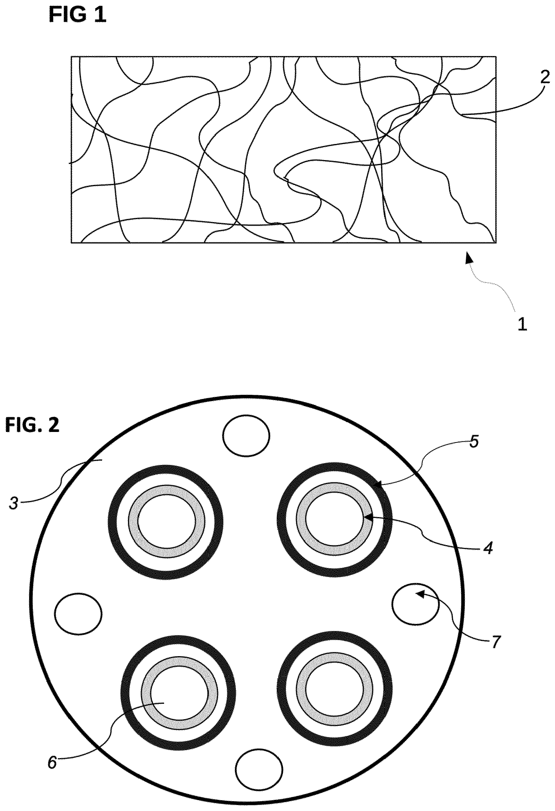

Cependant, l'implant ne se dégradant pas ou peu, le volume de l'implant ne doit pas gêner la reconstruction cellulaire. Il est particulièrement avantageux de former un implant de comblement qui comporte un support en hydrogel hétérogène hybride poreux avec des pores traversants qui autorisent la régénération cellulaire à l'intérieur de l'implant. Comme illustré à la figure 1, il est particulièrement avantageux que l'hydrogel hétérogène hybride 1 possède une structure poreuse dont les pores communiquent les uns aux autres pour créer un réseau percolant 2 dans les trois dimensions du volume du gel. Le réseau traversant formé par les pores permet aux cellules de migrer et de proliférer jusqu'au coeur de l'hydrogel, de se rejoindre et de survivre grâce au transport et à la diffusion des nutriments nécessaires au métabolisme cellulaire.

L'hydrogel étant non dégradable, les réseaux de pores n'induisent pas une dégradation accrue de l'hydrogel avec stagnation de molécules toxiques ou irritantes dans les pores.

L'implant est une structure poreuse avec des pores ouverts traversant l'implant pour définir des chemins percolants dans les trois dimensions de l'hydrogel. Les pores ouverts favorisent la colonisation cellulaire et vasculaire depuis le tissu jusqu'au coeur de l'implant ainsi que la circulation des fluides biologiques, des facteurs de croissance cellulaire, des facteurs de réparation cellulaire et des nutriments physiologiques à travers l'implant. Le passage des différents fluides à travers l'implant facilite la vascularisation du tissu généré dans l'implant. L'implant forme une matrice de support tridimensionnelle dont les pores traversants guident la croissance des cellules, des fibres nerveuses et des vaisseaux sanguins. La croissance cellulaire est de meilleure qualité en contact de l'implant que sans l'implant. L'implant peut être uniquement formé par l'hydrogel.

De manière préférentielle, la fraction poreuse de l'hydrogel formant l'implant est supérieure à 85%, plus préférentiellement au moins égale à 90% et encore plus préférentiellement au moins égale à 92% volumique. La fraction poreuse peut être calculée avec une technique de porosimétrie à mercure.

Il est préférable d'avoir un hydrogel hétérogène hybride dont la majorité de la fraction poreuse est formée par des pores dont le diamètre est compris entre 10 et 30 microns. Préférentiellement, au moins 60% de la fraction poreuse est formé par des pores ayant un diamètre compris entre 10 et 30 microns. En d'autres termes, au moins 60% du volume poreux est réalisé par des pores ayant un diamètre compris entre 10 et 30 microns.

Il est également intéressant d'avoir un hydrogel hétérogène hybride dont les pores ayant un diamètre compris entre 30 et 300 représentent une fraction plus importante que les pores ayant un diamètre inférieur à 10 microns. De manière préférentielle, la fraction des pores ayant un diamètre compris entre 30 et 300 microns est supérieure à 20% encore plus préférentiellement supérieure à 30%. La fraction des pores ayant un diamètre compris entre 30 et 300 microns est particulièrement intéressante pour loger des objets biologiques de grande taille comme des tissus multicellulaires. Avantageusement, la fraction des pores ayant un diamètre inférieur à 10 microns est inférieure à 15% encore plus préférentiellement inférieure à 10%. Il est encore avantageux que l'hydrogel hétérogène comporte moins de 2% volumique de pores ayant une taille inférieure à 1micron dans le réseau poreux traversant.

Une telle répartition dans les dimensions des pores assure le passage des composés biologiques à travers l'implant en couvrant tout le spectre dimensionnel des composés biologiques d'un tissu vivant. Cela facilite l'utilisation de l'implant en tant qu'équivalent tissulaire de l'organe à réparer.

Les pores de l'implant sont compatibles avec la circulation de chimiokines sécrétées par les cellules inflammatoires. La configuration de l'implant permet l'infiltration de cellules gliales, de cellules mésenchymateuses, de cellules souches/progénitrices associées à des leptoméninges sécrétrices du facteur SDF-1 et de cellules souches/progénitrices associées à l'épithélium du canal central ayant un potentiel de différenciation neuronale, l'infiltration des vaisseaux sanguins et la croissance de fibres nerveuses en régénération.

Il est également avantageux de former un hydrogel hétérogène hybride dans un matériau qui présente la propriété de changer la configuration de son réseau poreux au fur et à mesure de la colonisation cellulaire ou de la quantité de biocharge cellulaire. L'hydrogel hétérogène hybride peut se déformer sous la contrainte appliquée par la colonisation cellulaire et/ou la biocharge cellulaire. L'implant se déforme sous la pression exercée par le tissu cellulaire au fur et à mesure de la régénération. L'hydrogel doit pouvoir se déformer avec les contraintes mécaniques de la biocharge cellulaire, ce qui permet de garder une structure avec des canaux percolants.

Il est particulièrement avantageux de former un implant de comblement qui comporte un support en hydrogel hétérogène hybride poreux et déformable de manière viscoélastique et dont la vitesse de dégradation est suffisamment faible pour que l'hydrogel soit considéré comme un hydrogel hétérogène hybride non dégradable. Le volume occupé par l'implant poreux va évoluer au fur et à mesure de la régénération pour s'adapter en partie à la vitesse de régénération autour et dans l'hydrogel.

Lors de la régénération cellulaire et nerveuse, le squelette macromoléculaire de l'hydrogel hétérogène hybride se déforme à volume constant ou sensiblement constant. Lors de l'accumulation cellulaire dans l'implant et durant l'expansion du néotissu en formation, le réseau polymère se déforme sous la pression mécanique exercée par l'accumulation cellulaire. La structure de l'implant est réalisée dans un matériau qui possède un module élastique compris entre 1 et 200 KPa afin de s'adapter à la contrainte appliquée par la biocharge cellulaire. La valeur du module élastique peut être mesurée à 50% de sa déformation avant rupture.

Au fur et à mesure que la biocharge cellulaire augmente, l'implant se déforme en fonctions des contraintes mécaniques introduites par la biocharge cellulaire dans l'implant. L'implant se déforme élastiquement puis de manière viscoélastique afin de ne pas gêner la croissance cellulaire notamment dans les canaux traversants. Malgré sa déformation, l'implant conserve un réseau percolant tridimensionnel qui s'étire afin d'assurer une régénération tissulaire complète. Une fois le tissu reconstruit, le réseau polymère résiduel de l'implant sert de matrice intercellulaire de soutien qui stabilise le néotissu formé en exerçant une tension mécanique.

La surface de l'implant possède une rugosité importante qui permet une bonne adhésion avec le tissu hôte en augmentant la surface de contact entre l'implant et les cellules du tissu en contact, ce qui va favoriser l'adhésion entre les deux milieux. Les pores ouverts à la surface de l'implant et reliées au réseau percolant favorisent la colonisation cellulaire et vasculaire jusqu'au coeur de l'implant à partir du tissu en contact en même temps que la circulation des fluides biologiques, des facteurs de croissance cellulaire et des nutriments physiologiques nécessaires à la cellule.

Il est connu du document FR 2942408, l'utilisation d'un hydrogel hétérogène hybride pouvant former une matrice ayant des propriétés élastiques et une structure poreuse adaptée pour le traitement et la réparation d'organe et/ou la régénération tissulaire et notamment son utilisation comme bio matériau implantable. Il a été observé qu'un tel matériau possède une vitesse de dégradation inférieure à la vitesse de régénération d'un tissu cellulaire.

Il est particulièrement avantageux d'améliorer l'hydrogel hétérogène hybride connu du document FR 2942408 pour former un support non dégradable dont la structure poreuse définit spécifiquement des canaux percolants dans les trois dimensions et qui possède un comportement viscoélastique avec un module élastique compris entre 1 et 200kPa.

Il est avantageux d'utiliser un hydrogel hétérogène hybride qui est un copolymère dérivé d'au moins trois monomères : un monomère dendrimère fonctionnalisé par un radical éthylénique, un monomère acrylamide et un agent de réticulation. Dans une configuration particulière, l'hydrogel hétérogène hybride comporte un matériau copolymérisable bioactif. Dans une autre configuration particulière, l'hydrogel hétérogène hybride est dépourvu d'un matériau copolymérisable bioactif. La bioactivation peut être réalisée postérieurement sur l'hydrogel formé.

Le matériau copolymérisable bioactif est choisi parmi le groupe comportant les dérivés, d'un sucre complexe, d'un peptide d'adhérence à un tissu et d'un conjugué polymère couplé à des anticorps dirigés contre des dérivés lipidiques. Préférentiellement, le matériau copolymérisable bioactif est un dérivé méthacryloyle ou méthacrylamide d'un sucre complexe, d'un peptide d'adhérence à un tissu et d'un conjugué polymère couplé à des anticorps dirigés contre des dérivés lipidiques,

Le matériau copolymérisable bioactif peut être un dérivé, de préférence méthacryloyle ou méthacrylamide, d'un sucre complexe choisi, par exemple, parmi la glucosamine, la N-acétyl-glucosamine, la N-diglycidyl-glucosamine, la N-acétylgalactosamine, l'acide N-acétylneuraminique (acide sialique) et l'acide polysialique.

Le matériau copolymérisable bioactif peut être un dérivé, de préférence méthacryloyle ou méthacrylamide, d'un peptide d'adhérence à un tissu choisi parmi des oligopeptides d'adhérence à un tissu contenant des séquences d'acides aminés telles que Arg-Gly-Asp, Ile-Lys-Val-Ala-Val, Ala-His-Ala-Val-Ser-Glu, Tyr-Ile-Gly-Ser-Arg, des dérivés oligopeptides de molécules de différenciation tissulaire, par exemple, des protéines morphogénétiques osseuses ou de protéines de la famille SDF-1 (stromal cell derived factor-1), une chimiokine qui a la capacité de mobilisation et d'attraction des cellules souches endogènes qui expriment le récepteur CXCR4 et qui a la capacité de stimuler la croissance des axones lors de la régénération tissulaire.

Le matériau copolymérisable bioactif peut être un dérivé, de préférence méthacryloyle ou méthacrylamide, d'un conjugué polymère couplé à des anticorps contre la myéline et ses dérivés lipides associés aux axones.

Le monomère acrylamide est, avantageusement, un méthacrylamide N-substitué ou acrylamide N-substitué. La valeur du module élastique est en partie définie par la densité de réticulation de l'hydrogel, c'est-à-dire par le nombre de branchements covalents entre les chaines macromoléculaires formées à partir du monomère acrylamide, de préférence en contrôlant le nombre de branchements covalents entre les chaines macromoléculaires de HPMA.

Le méthacrylamide N-substitué est, de préférence, choisi parmi le groupe consistant en du N-monoalkylméthacrylamide, N,N-dialkylméthacrylamide, N-hydroxyalkylméthacrylamide, préférentiellement le N-(2-hydroxypropyl)méthacrylamide (HPMA), N-alkyl, N-hydroxyalkylméthacrylamide, et le N,N-dihydroxyalkylméthacrylamide.

L'acrylamide N-substitué est, de préférence, choisi parmi le groupe consistant en du N-monoalkylacrylamide, N-hydroxyalkylacrylamide, N,N-dialkylacrylamide, N-alkyl,N-hydroxyalkylacrylamide et N,N-dihydroxyalkylacrylamide.

Le monomère dendrimère comporte préférentiellement un noyau central A et des branches dendritiques macromoléculaires de poly(oxyéthylène) (PEO). Une seule des branches dendritiques est fonctionnalisée, avantageusement en position terminale, par un radical éthylénique. Le radical éthylénique est insaturé afin de réagir avec un monomère comportant au moins une double liaison vinylique réactive. Les autres branches dendritiques sont, de préférence, terminées par une fonction hydroxyle et dépourvues de radical éthylénique. Elles peuvent également être fonctionnalisées avant copolymérisation par d'autres fonctions comme des fonctions esters ou amide. Le radical éthylénique est, avantageusement, un radical acrylate ou méthacrylate. L'architecture en étoile du dendrimère permet d'introduire dans l'hydrogel une géométrie variable multifonctionnelle pour répondre de façon spécifique aux interactions multiples envisagées avec d'autres matériaux. L'indication selon laquelle une seule des branches est fonctionnalisée correspond à un résultat statistique indiquant qu'en moyenne, le monomère dendrimère est fonctionnalisé par une seule branche munie d'un radical éthylénique insaturé.

Les groupements fonctionnels hydroxyles dans l'hydrogel hétérogène hybride peuvent être modifiés en attachant des entités, par exemple, des agents bioactifs tels que des polypeptides, des principes actifs, des ligands, des groupements polymérisables ou des oligosaccharides.

Selon un mode de réalisation particulier, le noyau central A est un groupement choisi parmi un carbosilane, un polycarbosilane, un polycarbosilane à architecture d'étoile, ou un groupement répondant à la formule (1) suivante :

-(CH₂)ₙ-Si-(CH₂)ₙ- (1)

où n est un entier compris entre 1 et 20, de préférence égal à 6.

Selon une variante, le noyau central A est un poly(divinylbenzène).

Selon un mode de réalisation préférentiel, le monomère dendrimère répond à la formule (2) suivante :

(CH₂=CR-CO-O-(CH₂CH₂O)_{Z})_{Y}-A-(O-(CH₂CH₂O)_{Z'}-H)_{X} (2)

Dans laquelle,
R est H ou CH₃,
X et Y sont des entiers compris entre 1 et 100 et la somme X+Y est un multiple de 4, et
Z et Z' sont identiques ou différents et compris entre 1 et 100, et
A étant, de préférence, un carbosilane, un polycarbosilane, un polycarbosilane à architecture d'étoile, ou un groupement répondant à la formule (1).

Selon un autre mode de réalisation préférentiel, le monomère dendrimère répond à la formule (3) suivante :

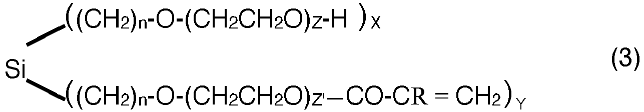

Dans laquelle :
R est H ou CH₃,
n étant un entier compris entre 1 et 20, de préférence égal à 6,
Z et Z' sont identiques ou différents et compris entre 1 et 100,
X est égal à 1, 2 ou 3 et,
Y répond à la formule Y=4-X.

Avantageusement, le monomère dendrimère est fonctionnalisé par un radical méthylméthacrylate et possède quatre branches dendritiques de PEO et un noyau central A silane à pont hexanol c'est-à-dire ayant une structure de formule (3) avec R=CH₃, n=6, X=3, Y=1, et Z=Z'. Pour des raisons de clarté, nous identifierons ce monomère dendrimère par la notation Si-PEO4-MMA.

Les propriétés mécaniques et chimiques de l'hydrogel peuvent également être ajustées en greffant, sur les terminaisons hydroxyles libres des branches dendritiques, des fonctions aux propriétés spécifiques telles que des propriétés hydrophiles, hydrophobes et/ou tensioactives. La fonction hydroxyle est facilement fonctionnalisable du fait du caractère nucléophile de son atome d'oxygène. Cette fonction est connue pour être facilement activée, par exemple, par traitement basique. Les propriétés intrinsèques de l'hydrogel hétérogène hybride peuvent également être modifiées en faisant varier le nombre de monomères dendrimères introduits dans le squelette hydrogel et la nature du noyau central A. Le nombre de branches dendritiques conditionne la solubilité de la macromolécule dans l'eau et les solvants organiques.

L'hydrogel hétérogène hybride est formé majoritairement ou est constitué par une pluralité de microbilles qui sont assemblés les unes aux autres pour définir le réseau poreux traversant. Les microbilles présentent une forme sphérique ou sensiblement sphérique et majoritairement un diamètre supérieur à 1,5 microns et inférieur à 10 microns. De manière préférentielle, les microbilles présentent majoritairement un diamètre compris entre 2 et 5 microns pour mieux moduler la déformation des canaux selon la contrainte appliquée par la biocharge cellulaire.

De manière à mieux contrôler la conformation du réseau percolant tridimensionnel, il est préférable d'agglomérer les microbilles d'hydrogel sous la forme d'agrégats qui comportent au moins 5 microbilles et moins de 50 microbilles et préférentiellement au moins 10 microbilles. De manière avantageuse, les microbilles s'agglomèrent entre elles pour former des agrégats par groupes de 10 à 30 microbilles. Il est également particulièrement avantageux que les microbilles d'hydrogel s'agglomèrent entre elles selon une configuration dite en « grappe de raisins » ce qui facilite la formation d'un réseau poreux efficace tout en assurant une bonne déformabilité du support. Il est préférable que les agrégats soient formés par des microbilles dont le diamètre soit compris entre 5 et 10 microns. Les agrégats forment préférentiellement le réseau primaire de la matrice hydrogel. Les agrégats sont fixés les uns aux autres par des points de fixation qui autorisent le déplacement d'un agrégat par rapport à un autre. Dans une conformation en grappe de raisin, la section évolue de manière croissante depuis une extrémité à l'autre et la section est sensiblement circulaire perpendiculairement à la longueur de l'agrégat. La conformation en grappe de raisin est plus avantageuse que la configuration linéaire pour faciliter les interactions entre les cellules et l'implant et ainsi faciliter la formation d'une structure tissulaire multicellulaire et/ou la croissance des fibres nerveuses. La conformation en grappe permet également d'améliorer l'adhérence

Dans un agrégat, les microbilles sont fixées les unes aux autres par des points de réticulation déformables pour autoriser les déplacements des microbilles entre elles et adapter la conformation du support en hydrogel hétérogène hybride selon les contraintes mécaniques appliquées. L'agrégat peut se compresser. Les chaines polymères se déforment entre les points de réticulation en s'étirant. Les points de réticulation s'écartent les uns des autres ce qui se traduit par une augmentation de la fraction poreuse de l'hydrogel. Lors de la régénération cellulaire et nerveuse, le volume poreux total de l'implant augmente à volume constant selon une configuration anisotropique.

Les agrégats de microbilles sont montés mobiles les uns par rapport aux autres de manière à autoriser la contraction des agrégats de microbilles en réponse à la pression capillaire exercée par les cellules migrantes en contact avec la surface des microbilles. Les agrégats de microbilles sont configurés de sorte que leur contraction a pour effet une expansion du réseau poreux et une augmentation du volume poreux disponible à l'expansion du tissu en formation.

Les agrégats sont fixés les uns aux autres pour former un réseau poreux comprenant des domaines mésoporeux et macroporeux. Les multiples pores définis par les agrégats sont connectés entre eux pour former un réseau percolant qui présente une certaine tortuosité et qui traverse l'hydrogel dans sa configuration initiale. Au niveau microscopique, le réseau percolant permet l'infiltration des cellules, des vaisseaux sanguins et des fibres nerveuses à travers l'hydrogel dans les trois dimensions de l'espace lors du processus de bioreconstruction du tissu. En parallèle, les microbilles définissent des micropores dont la taille est inférieure à 20nm, de préférence dont la taille varie dans l'intervalle de 1,5 et 11 nanomètres avec un diamètre moyen de 6 nanomètres. Ces micropores sont fermés et non communicants. Les micropores forment des cratères à la surface des microbilles, ce qui confère aux microbilles d'hydrogel une surface alvéolée. Ces micropores créent de la surface et contribuent à accroître la surface spécifique interne du réseau macromoléculaire de l'hydrogel formée de ces microbilles d'hydrogel. Cette augmentation de la surface du réseau macromoléculaire et la topographie de surface des microbilles favorisent les interactions avec les membranes cellulaires notamment les adhésions focales des cellules en migration.

Préférentiellement, le réseau percolant définit une surface spécifique interne importante, au moins égale à 25m²/g, avantageusement au moins 50m²/g.

Dans l'art antérieur, à cause de la dégradation de la matrice hydrogel, il a été observé que la masse de biocharge cellulaire augmente avec le temps et remplit en partie le volume perdu par la matrice hydrogel. Il ressort également que la vitesse de dégradation de l'hydrogel est supérieure à la vitesse de colonisation cellulaire ce qui complique la répartition de la biocharge dans le volume initialement occupé par l'implant. Au contraire, avec un hydrogel hybride non-dégradable, poreux et déformable de manière viscoélastique, la biocharge s'accroit par unité de volume du défaut tissulaire en profitant de la compression et de la déformation de l'hydrogel. La colonisation cellulaire est mieux maitrisée. Le réseau macromoléculaire déformable évolue dans le temps avec la croissance des cellules qui s'organisent, migrent et se différencient de manière autonome pour former un néotissu fonctionnel.

Il est particulièrement avantageux de former un implant dont la densité de réticulation est inhomogène. La différence de degré de réticulation peut être définie au moyen du procédé de fabrication utilisé. Il est préférentiel d'utiliser une copolymérisation de l'hydrogel au moyen d'une méthode qui met en oeuvre une séparation de phase du mélange réactionnel, il est également avantageux de mettre en oeuvre un procédé de copolymérisation dont la séparation de phase provient d'une polymérisation induite par voie thermique. Il est particulièrement avantageux de contrôler la séparation de phase en réalisant la réticulation dans une gamme de température définie. Par exemple, de bons résultats ont été obtenus avec une séparation de phase comprise entre en 40°C et 60°C. Un support plus performant a été obtenu avec une réticulation comprise entre 45°C et 55°C. Un support encore plus performant a été obtenu avec une réticulation comprise entre 49°C et 51°C. L'utilisation d'une telle gamme de température permet de mieux définir les dimensions des microbilles.

Il est également avantageux de limiter la vitesse de polymérisation pour assurer la formation d'un hydrogel associant les bonnes performances mécaniques et les canaux traversants. De manière préférentielle, la durée de réticulation est supérieure à 6 heurs voire supérieure à 12 heures pour former un implant.

Il est particulièrement intéressant de réaliser un implant dans lequel les agglomérats sont des régions du réseau hydrogel où la densité de réticulation est élevée. L'hydrogel est formé par des agrégats fortement réticulés liés entre eux par des zones faiblement réticulées. Les agrégats fortement réticulés forment des zones de support mécaniques qui peuvent se déplacer les unes par rapport aux autres au moyen des zones faiblement réticulées.

Les points de réticulations entre les agrégats sont formés par des liaisons covalentes, c'est-à-dire des liaisons chimiques que l'on peut considérer comme non dégradables ce qui permet une bonne tenue mécanique entre les agrégats. Il est avantageux de prévoir que les agrégats soient répartis de façon statistique dans le volume de l'hydrogel définissant des domaines de forte concentration de chaînes polymériques et des domaines de faible concentration de chaînes polymériques et formant des cavités contenant l'eau libre.

La distance moyenne entre deux points de réticulation dans les zones fortement réticulées est inférieure à 20% de la distance moyenne entre deux points de réticulation dans les zones faiblement réticulées, de préférence inférieure à 10% de la distance moyenne entre deux points de réticulation dans les zones faiblement réticulées.

Il est préférable que la proportion de la zone à forte réticulation représente au moins 60% du volume total d'intrusion de l'hydrogel. Il est également intéressant que la proportion de la zone à forte réticulation représente moins de 80% du volume total d'intrusion de l'hydrogel.

La surface de l'implant n'est pas plane. Elle présente des défauts par exemple des protubérances provenant de l'assemblage des microbilles.

Il est particulièrement avantageux de réaliser un hydrogel hétérogène hybride dans lequel les microbilles comportent au moins 90% en poids voire au moins 95% en poids ou sont constituées du composé acrylamide, par exemple de méthacrylamide N-substitué ou acrylamide N-substitué pour conférer des propriétés élastiques aux microbilles assurant la déformation de l'implant avec la charge cellulaire. De manière particulière, les microbilles majoritairement ou exclusivement en composé acrylamide possèdent un diamètre compris 1,5 et 10 microns de préférence entre 2 et 5 microns.

De manière préférentielle, les microbilles contiennent majoritairement en masse du HPMA, de préférence du HPMA fortement réticulé. De manière avantageuse, une microbille fortement réticulée possède un taux de réticulation supérieur à 1 mol% de réticulant. L'utilisation d'un tel taux de réticulation assure l'existence d'un nombre suffisant de liaisons transversales chimiques entre les chaines de polymères linéaires par unité de volume de l'hydrogel. Cette configuration confère à l'hydrogel une cohésion suffisante des réseaux polymériques, par exemple le HPMA, pour un taux de gonflement à l'équilibre de 96% de la masse finale (g d'eau/g de matière sèche). Dans le cas présent ce taux est préférentiellement de 0,95 mol%. Du matériau plus faiblement réticulé (par exemple le HPMA) peut être utilisé pour former les liaisons entre les microbilles et définir les agrégats de microbilles.

Il est particulièrement avantageux de prévoir que les canaux traversants de l'implant soient délimitées par des microbilles dont le monomère dendrimère fonctionnalisé comporte une ou plusieurs branches dendritiques macromoléculaires de poly(oxyde d'éthylène). La branches dendritiques macromoléculaires de poly(oxyde d'éthylène) est fonctionnalisée par une ou plusieurs molécules active, par exemple un des matériaux copolymérisable bioactifs décrits plus haut. Il est alors possible de fonctionnaliser la surface du réseau percolant pour faciliter la reconstruction cellulaire à l'intérieur de l'implant.

La molécule active est choisie parmi le groupe consistant en un dérivé de sucres complexes, de dérivés de peptide d'adhérence à un tissu ou de peptide avec une activité angiogénique, de dérivés de peptide de stimulation de la repousse nerveuse, de dérivés de peptide de stimulation de la prolifération et la différenciation cellulaire, un dérivé d'un conjugué polymère couplé à des anticorps dirigés contre des dérivés lipidiques, des chimiokines de la classe des Stromal-derived factor-1 (SDF-1).

Il est également avantageux de prévoir d'utiliser des microbilles dans lesquelles le composé acrylamide est le N-(2-hydroxypropyl) méthacrylamide (HPMA) et le monomère dendrimère fonctionnalisé par un radical éthylénique comporte des dendrimères de poly(oxyde d'éthylène) fonctionnalisés à leur périphérie avec des agents bioactif conférant des propriétés bioactives pour la régénération tissulaire.

Comme pour le document FR 294208, un hydrogel hétérogène hybride peut être réalisé au moyen d'une copolymérisation radicalaire. Le procédé de polymérisation préférentiel est effectué en milieu polaire organique et avantageusement bi-organique, par exemple un mélange acétone/DMSO.

De manière avantageuse, en plus de la copolymérisation radicalaire, la copolymérisation est associée à une séparation de phases induite par polymérisation (PIPS). De cette manière, la solution initialement homogène de monomères et de solvants polaires se sépare en cours de la copolymérisation pour former les microbilles. Avantageusement, le rapport molaire entre le monomère dendrimère fonctionnalisé et l'agent de réticulation est compris entre 0,1 et 0,8, par exemple un rapport PEO-MMA/MbisAA compris entre 0,1 et 0,8. Avec cette valeur de rapport, la séparation de phase définit des pelotes qui forment les microbilles. Dans la gamme de température 45-55°C, il est plus facile d'obtenir majoritairement par des pelotes en composé acrylamide dont le diamètre est compris entre 1,5 et 10 microns. Cette valeur de rapport permet également la formation d'un réseau percolant tridimensionnelle. Il est encore plus facile de contrôler le nombre de pelotes générées et la distribution en taille des pelotes en choisissant un monomère dendrimère fonctionnalisé dont la masse moléculaire est comprise entre 6220g/mol et 23280g/mol. Dans cette gamme particulière lorsque la masse molaire du monomère dendrimère fonctionnalisé augmente, le nombre de pelotes augmente et la distribution en taille diminue.

Le procédé de formation de l'hydrogel hétérogène hybride comporte une première phase de la réaction du mélange réactionnel formants des nucléi insolubles constitués de chaines de polymères conformées en pelote. Comme illustré à la figure 2, le mélange réactionnel est injecté dans des moules 3 de forme cylindrique étanche et conducteur de la chaleur avec une paroi 4 en PTFE en contact avec le mélange réactionnel. Le mélange réactionnel peut avoir subi un dégazage avec de l'argon.

Les microbilles sont associées pour former les agrégats et les agrégats sont liés entre eux pour former l'hydrogel hétérogène hybride destiné à former l'implant de comblement.

Il est également possible de prévoir la fourniture d'agrégats de microbilles et l'assemblage des agrégats les uns aux autres par un procédé d'impression tridimensionnelle pour former l'implant de comblement. La forme de l'implant est directement définie par l'équipement d'impression tridimensionnelle.

La copolymérisation est réalisée au moyen d'un agent de réticulation. L'agent de réticulation peut être un acrylamide comme du bisacrylamide de méthylène (MbisAA), des précurseurs de celui-ci ou des composés divinyles tels que le divinylbenzène (DVB). L'initiateur de polymérisation à radicaux libres est sélectionné parmi les initiateurs connus comme l'azobisisobutyronitrile (AIBN) ou le peroxyde de benzoyle.

L'agent de réticulation est préférentiellement du bisacrylamide de méthylène (MbisAA) qui possède deux groups vinyles. Le bisacrylamide de méthylène permet une réactivité supérieure en comparaison du N-(2-hydroxypropyl)méthacrylamide (HPMA) qui ne possède qu'un seul groupe vinyle. Cela permet une incorporation plus rapide du bisacrylamide de méthylène dans les chaines croissantes qui forment des nucléi avant de former les microbilles.

Il est particulièrement intéressant d'utiliser un hydrogel du poly(oxyde d'éthylène) car le poly(oxyde d'éthylène) a démontré un effet neuroprotecteur sur la membrane des neurones et diminue le stress oxydatif post-lésionel (Luo et al. Polyethylene glycol immediately repair neuronal membranes and inhibits free radical production after spinal cord injury. J. Neurochem. 83, 471, 2002)*.* Il est préférable d'utiliser un hydrogel formé à partir d'un ou plusieurs polymères à structure ramifiée en étoile avec des branches de poly(oxyde d'éthylène) ce qui améliore la biocompatibilité de l'hydrogel notamment en empêchant l'absorption non spécifique des protéines du sang, ce qui module la réponse inflammatoire locale en diminuant l'activation du complément par le clivage de la protéine C3 en peptides responsables du recrutement des cellules phagocytaires (Nilsson, B., et al. The rôle of complément in biomaterial-induced inflammation. Mol Immunol 44, 82, 2007)*.*

Ces molécules ramifiées peuvent être modifiées avec des groupements fonctionnels de la classe des peptides, des sucres bioactifs, des chimiokines par bioconjugaison en position terminale des branches de poly(oxyde d'éthylène). Il est particulièrement avantageux de former un matériau hydrogel qui possède une structure poreuse réalisée dans un support comportant une structure composée du N-(2-Hydroxypropyl) methacrylamide et une structure ramifiée formée par des polymères de poly(oxyde d'éthylène) ramifiés porteurs de groupements fonctionnels. Les groupements fonctionnels sont préférentiellement des peptides courts tels que des oligopeptides d'interaction avec les récepteurs intégrines des cellules, par exemple, mais non limité aux séquences suivantes, Arg-Gly-Asp, (RGD), Arg-Gly-Asp-Ser (RGDS), Ile-Lys-Val-Ala-Val (IKVAV). D'autres groupements fonctionnels peuvent être des oligomères conjugués à un ou plusieurs acides sialiques, par exemple du sialyllactose (Neu5Ac-a2,3-Gal-β1,4-Glc), ainsi que des oligosaccharides sulfatés comme le HNK1 (SO₄-3-GlcAβ1-4Galβ1-4GlcNac-R) ou des oligosaccharides fucosylés, par exemple le Fuc a(1-2)Gal. Des agents bioactifs peuvent aussi être choisis parmi des facteurs de croissance qui stimulent la régénération des axones qui inclut, mais non limité au facteur BDNF ("Brain Derived Neurotrophic Factors"), IGF-1 ("Insuline-like Growth Factor"), NT-3 (« Neurotrophin »), GDNF (« Glial Derived Neurotrophis Factor ») ; ou qui stimule la prolifération de précurseurs neuronaux comme le FGF ("Fibroblast Growth Factors"), et le EGF ("Epidermal Growth Factor). Le facteur PDGF ("Platelet-derived Growth Factor"), VEGF ("Vascular Endothelial Growth Factor"), PIGF ("Placental Growth Factor"), NGF ("Nerve Growth Factor") et le TGF ("Transforming Growth Factor") peuvent être conjugués au POE.

Des agents bioactifs qui stimulent la mobilisation de cellules souches précurseurs comme les chimiokines SDF-1 (*Stromal-derived factor-1*) qui ont la capacité d'attraction des cellules souches endogènes exprimant le récepteur CXCR4 et de stimuler la croissance des axones lors de la régénération tissulaire, les cytokines G-CSF (*granulocyte colony-stimulating factor)*, GM-CSF (*granulocyte-macrophage colony-stimulating factor)* ou SCF (*Stem cell factor*) et l'interleukine (IL-8).

Un mode de réalisation de l'hydrogel est réalisé avantageusement sous atmosphère inerte et comprend un hydrogel qui combine le N-(2-hydroxypropyl) méthacrylamide à l'agent de réticulation N,N'-Méthylènebisacrylamide ou le N,N'-Méthylènebismethacrylamide comprenant deux groupes vinyle dans un rapport molaire de 100:1 pour 30,4% en poids du poids total du mélange réactionnel ; un solvant polaire bi-organique acétone/DMSO (93/7 v/v).

Le mélange réactionnel est dégazé avec de l'argon et injecté dans des moules de forme cylindrique étanche et conducteur de la chaleur avec une paroi en PTFE en contact avec le mélange réactionnel. De manière avantageuse, le mélange réactionnel initial est disposé dans un moule en métal de préférence en acier inoxydable dont les parois internes sont recouvertes d'un polytétrafluoroéthylène (PTFE). Le mélange réactionnel est purgé avec un gaz inerte directement à l'intérieur du moule. Préférentiellement, le moule possède une section circulaire avec une hauteur au moins deux fois supérieure au diamètre. L'utilisation d'un moule métallique facilite son utilisation avec un bain marie qui fixe la température du moule et du mélange réactionnel durant la polymérisation. Cette configuration permet une meilleure maitrise des dimensions des microbilles et des pores.

Une réaction de polymérisation radicalaire préférentiellement en présence de l'amorceur azobisisobutyronitrile à 50°C est réalisée. Il a été observé qu'il est particulièrement avantageux d'augmenter la température du moule et du mélange réactionnel d'au moins 5°C, de préférence de 10°C ou plus en cours de polymérisation. Il est avantageux que l'augmentation de température soit inférieure à 20°C. Il est également avantageux que la température maximale de la polymérisation soit inférieure à 70°C et encore plus préférentiellement inférieure à 65°C pour ne pas dégrader le xérogel, c'est-à-dire l'hydrogel avant sa saturation en eau. Cette augmentation de la température permet d'obtenir une distribution plus homogène des chaines polymériques dans l'hydrogel ainsi qu'un meilleur rendement. De manière préférentielle, l'augmentation de température est réalisée après la formation des microbilles ou d'une majorité des microbilles. L'augmentation de température peut intervenir au moins après 80 minutes de polymérisation, encore plus préférentiellement après 90 minutes de polymérisation. La polymérisation peut être réalisée avec un premier plateau de température utilisé pour former les microbilles, par exemple un plateau de température compris entre 45°C et 55°C de préférence égal à 50°C. Le plateau de température est ensuite suivi par un deuxième plateau ou éventuellement par une rampe ou une autre forme de recuit à une température au moins 5°C supérieure à la température du plateau. L'utilisation de deux gammes des températures différentes permet une meilleure maitrise des dimensions des microbilles et une meilleure maitrise des dimensions des agrégats. De préférence, durant le premier plateau en température, la polymérisation est réalisée jusqu'à ce que la concentration en oligomères atteint une valeur seuil permettant de former des chaines plus longues par condensation des oligomères. La condensation des oligomères induit l'apparition d'au moins deux phases ayant des densités différentes. Il est avantageux de réaliser une séparation de phase en modifiant la température de polymérisation.

Pour détecter que la concentration seuil en oligomères est atteinte, il est avantageux de suivre le signal d'absorbance de la réaction. Par exemple, on suit un signal d'absorbance en spectrométrie d'absorption (densité optique) dans le domaine ultraviolet-visible. La détection d'une quantité suffisante en chaines longues obtenues par condensation des oligomères peut correspondre à une valeur seuil d'absorbance ou une vitesse d'évolution de l'absorbance qui atteint une valeur seuil. Une fois que l'on détecte la valeur seuil, on sait que les chaines longues sont en quantité suffisante. Une deuxième étape de polymérisation est réalisée à plus haute température, préférentiellement avec un deuxième plateau qui est au moins plus chaud de 5°C que le plateau précédent. Le deuxième plateau permet de réaliser la copolymérisation en accélérant la nucléation et en permettant la poursuite de la polymérisation. Il se forme des pelotes d'oligomères réticulés qui forment les microbilles. Les microbilles s'agrègent aléatoirement et les points de réticulation se forment. Durant la deuxième phase de polymérisation à une plus haute température, la valeur de l'absorbance décroit. En alternative, le deuxième plateau est remplacé par une rampe ou une évolution plus complexe de la température et qui possède une température minimale supérieure à la température du premier plateau d'au moins 5°C.

Durant la polymérisation, il est préférable de fermer le moule 3 de manière étanche, par exemple au moyen d'un couvercle en polytétrafluoroéthylène avantageusement associé à un joint 5. L'utilisation du couvercle limite l'évaporation des solvants à partir du mélange réactionnel et permet une meilleure reproductibilité.

De manière préférentielle, les dimensions du moule sont choisies de sorte que l'hydrogel formé à partir du moule présente un diamètre égal à 175mm et une hauteur égale à 400mm pour un gel hydraté. Il est également avantageux d'utiliser une même pièce métallique qui définit plusieurs moules sous la forme de plusieurs puits 6. Le moule 3 peut comporter des orifices 7 destinés à coopérer avec des vis pour fermer les puits 6.

A la fin de la polymérisation, le xérogel se trouve sous une forme sèche ou anhydre et est démoulé. Il est particulièrement avantageux de recouvrir les parois internes du moule avec du polytétrafluoroéthylène car cela permet un démoulage plus facile de l'hydrogel sous forme sèche ce qui évite de l'abimer. Le xérogel présent dans le moule est friable ce qui en fait un matériau fragile qui est facilement dégradé lorsque l'on cherche à l'extraire des ampoules utilisées dans les procédés de l'art antérieur.

Un lavage du xérogel dans éthanol/eau apyrogène est réalisé ce qui lui permet d'atteindre un taux de gonflement à l'équilibre de 96%. En alternative, l'éthanol est remplacé par du méthanol. De manière avantageuse, le xérogel est transféré dans un premier récipient se présentant sous la forme d'un panier ajouré, le panier est avantageusement en polytétrafluoroéthylène. De manière préférentiellement le premier panier est installé dans un second panier qui contient un liquide. Le liquide peut être de l'eau, de l'éthanol ou du méthanol ou un mélange d'eau avec de l'éthanol ou du méthanol. Le liquide du deuxième récipient passe à travers les trous du premier récipient pour nettoyer le xérogel.

Il est particulièrement avantageux que le deuxième récipient soit opaque au rayonnement visible. Le deuxième récipient peut être en polycarbonate.

Le xérogel installé dans le premier récipient est soumis à un cycle de lavage qui comporte avantageusement l'application de plusieurs bains de lavage successifs. La teneur en eau est croissante dans les différents bains afin de nettoyer le xérogel et de le saturé en eau pour former l'hydrogel.

L'hydrogel est avantageusement réticulé avec le méthylènebisacrylamide à un taux égal ou sensiblement également égal à 1 mol% qui confère à l'hydrogel des caractéristiques physiques avantageuses comme implant de comblement.

Pour son utilisation en chirurgie comme implant de comblement pour être manipulé dans des conditions aseptiques, il est nécessaire que la stérilité du produit soit assurée. Le gel est placé avantageusement dans un contenant cylindrique en PTFE de haut grade, un matériau qui n'interagit pas avec la nature chimique du gel, et rempli avec de l'eau de grade injectable apyrogène. Le contenant en PTFE avec le gel est stérilisé par autoclavage à 121°C pendant 30 minutes et le contenant est refermé avec un couvercle à vis de façon étanche pour que le gel reste saturé en eau et stérile. Ce contenant est placé dans un deuxième contenant en polystyrène avec un « cap snap safe type » qui est ouvert sur un champ stérile opératoire. Ainsi le contenant en PTFE peut être manipulé stérilement. Le deuxième contenant en polystyrène peut présenter une hauteur égale à 54mm, un diamètre interne égale à 34mm et une épaisseur égale à 1,5mm.

L'hydrogel hétérogène hybride est avantageusement utilisé dans une stratégie de comblement afin de modifier les phénomènes de cicatrisation naturelle en réalisant le comblement par un phénomène de régénération en présence d'une matrice viscoélastique qui a la propriété de changer la configuration de son espace géométrique poreux et la configuration de son réseau polymères. Les effets d'élasticité de l'hydrogel offrent l'avantage de pouvoir modifier les propriétés mécano-élastiques du substrat et reconstituer un environnement mécanique proche de celui des cellules in vivo. Un tel hydrogel est apte à recevoir et à guider des flux de cellules, les fibres nerveuses et les vaisseaux sanguins.

L'hydrogel hétérogène hybride définit un milieu poreux élastiquement déformable et continu, non dégradable et non biorésorbable avec une géométrie qui s'adapte à la cinétique de régénération cellulaire, vasculaire et nerveuse. Une fois implantée dans une zone incisée, la matrice hydrogel s'arrime à la moelle épinière ce qui permet à la matrice hydrogel de suivre les mouvements de la moelle épinière engendrés par les mouvements de la colonne vertébrale et par les battements des artères qui irriguent cet organe et donc de rester ancrer au site de greffe. L'implant forme une structure support, grâce à sa structure poreuse ouverte qui permet au flux de cellules de migrer dans la matrice de polymères en suivant les chemins percolants du réseau poreux.

Dans un mode de réalisation particulier, l'hydrogel hétérogène hybride forme un implant dans une cavité de greffe, par exemple une cavité intramédullaire. La cavité peut être formée par dissection et élimination de tissus cicatriciels non vivants à partir d'un bord interne de la lésion. Les bords de la cavité sont formés par un tissu nerveux sain. Dans un mode de réalisation avantageux, la fourniture de la cavité est suivie du drainage du liquide céphalo-rachidien. Cela permet de créer une lésion « de novo » qui équivaut à une lésion aiguë ce qui réactive les processus inflammatoires de réparation cellulaire endogène (mobilisation de cellules souches, bourgeonnement des extrémités nerveuses, angiogenèse). La méthode d'implantation comprend également une étape de comblement de la cavité post-traumatique avec l'introduction de l'hydrogel dans la cavité intra médullaire. L'hydrogel peut être découpé pour s'ajuster à la forme et à la géométrie de la cavité.

Il est particulièrement avantageux de déshydrater partiellement l'hydrogel avant de l'introduire dans la cavité. Après installation dans la cavité, l'hydrogel gonfle suite à la mise en contact avec les fluides circulant dans la cavité jusqu'à ce que l'implant d'hydrogel se trouve en contact avec la totalité de la surface de la cavité de telle sorte qu'il forme une interface intégrale (100%) avec la substance blanche du tissu nerveux péri lésionnel intact. L'hydrogel est capable d'absorber une quantité importante d'eau et il gonfle en présence d'eau et de liquides biologiques contenant de l'eau. Il est avantageux que l'hydrogel contienne au moins 80% volumique d'eau à l'équilibre. Il est avantageux de fournir l'hydrogel avec une teneur en eau inférieure ou égale à 75% volumique. Préférentiellement, l'implant est déshydraté de manière à avoir une réduction de son volume comprise entre 10% et 30%. Il est également avantageux de réaliser la déshydratation des surfaces de la cavité, par exemple les surfaces parenchymateuses. Il est préférable de déshydrater les parois avec une éponge ophtalmique. Ensuite, l'implant est introduit dans la cavité, de préférence, une cavité intra-parenchymateuse, puis il est réhydraté pour atteindre au moins 95% de son volume initial, de préférence 100% de son volume initial. L'hydratation de l'implant pour atteindre son volume initial peut être réalisé en moins d'une minute. De manière préférentielle, le volume de l'implant avec un taux de gonflement égal à 100% représente entre 80% et 100% du volume de la cavité à remplir. Une fois l'implant réhydraté, les surfaces poreuses de l'implant viennent en contact des surfaces de la cavité ce qui facilite l'adhésion. Il est avantageux d'avoir un gradient dans le taux de déshydratation avec une portion centrale plus hydratée que la portion périphérique. Une déshydratation plus importante à la surface permet d'améliorer la qualité du contact avec la paroi de la cavité par la suite.

Pour favoriser l'adhésion de l'implant avec les parois de la cavité, il est préférable de déshydrater puis d'hydrater les parois destinées à venir en contact. Il est également préférable d'avoir un implant qui se présente sous la forme d'une pluralité de grappes de raisin formés par des microbilles. La conformation de l'implant avec les microbilles précitées permet de définir une rugosité de surface avec des aspérité et des protubérances. Il est avantageux d'avoir une rugosité inférieure ou égale à 30 micromètres de préférence inférieure ou égale à 15 micromètres et encore plus avantageusement inférieure ou égale à 5 micromètres. Il est également avantageux que la rugosité soit supérieure ou égale à 0,1 micromètre. Une telle gamme de rugosité permet de favoriser la circulation des fluides interstitielles entre la surface de l'hydrogel et le parenchyme. Cette texture de surface provient de la structure en grappe des microbilles de l'hydrogel. L'adhésion est aussi favorisée par les pores de surface sur les protubérances sur une échelle nanométrique.

Il est particulièrement avantageux de recouvrir la surface de l'hydrogel avec de la substance blanche péri lésionnelle vivante comprenant les fibres nerveuses ascendantes, descendantes et d'association. L'implant est particulièrement adapté pour épouser la géométrie des surfaces de la cavité. Ces étapes aboutissent à la reconstitution anatomique de la moelle épinière au niveau de sa zone traumatique. Il est particulièrement avantageux de recouvrir l'implant avec de la substance blanche péri lésionnelle vivante comprenant les fibres nerveuses ascendantes, descendantes et d'association pour profiter de propriétés bioadhésives de l'implant avec le tissue biologique.

Afin d'améliorer la qualité de la reconstruction, il est avantageux d'avoir un implant qui possède des propriétés bioadhésives ou des propriétés bioadhésives améliorées. En adaptant les propriétés de la surface du l'implant, cela améliore l'adhésion entre l'implant et le tissue biologique.

Lorsque l'implant possède des propriétés bioadhésives améliorées cela permet de l'insérer dans la cavité et d'avoir une reconstruction cellulaire de qualité sans utiliser de suture chirurgicale avec le tissu biologique.

Il est particulièrement avantageux que l'implant stimule activement l'hémostase et plus particulièrement la phase primaire de l'hémostase. Il est également avantageux que l'implant stimule activement l'agrégation plaquettaire. De cette manière, l'implant induit la coagulation sanguine ce qui facilite la réalisation d'une bonne reconstruction tissulaire. Cette configuration particulière d'implant permet de contrôler les microsaignements au plus près de l'interface entre l'implant et la cavité et ce qui évite la formation d'une interface dégradée entre l'implant et le tissu. Des tests in vitro de coagulation sanguine et d'agrégation plaquettaire ainsi que des tests in vivo ont montré le bon contrôle de l'hémostase par l'implant.

## Revendications

1. Hydrogel hétérogène hybride formé au moyen d'un copolymère dérivé d'au moins trois des monomères suivants :
- un monomère dendrimère fonctionnalisé par une seule branche munie d'un radical éthylénique insaturé,
- un composé acrylamide choisi parmi un méthacrylamide N-substitué et un acrylamide N-substitué, et
- un agent de réticulation,
dans lequel l'hydrogel hétérogène hybride est formé majoritairement par une pluralité de microbilles ayant un diamètre supérieur à 1,5 microns et inférieur à 10 microns et contenant majoritairement en poids le méthacrylamide N-substitué et l'acrylamide N-substitué,
dans lequel les microbilles sont assemblées pour définir un réseau poreux traversant définissant des chemins percolants tridimensionnels,
dans lequel le réseau poreux traversant définit des pores dont la majorité de la fraction poreuse est formée par des pores dont le diamètre est compris entre 10 et 30 microns,
hydrogel hétérogène hybride **caractérisé en ce que** les microbilles définissent des micropores fermés et non communicants dont la taille est inférieure à 20nm, les microbilles étant assemblées les unes aux autres pour former des agrégats contenant entre 5 et 50 microbilles, les agrégats présentant une densité de réticulation plus importante que des zones plus faiblement réticulées qui permettent le déplacement des agrégats les uns par rapport aux autres,
dans lequel la fraction des pores ayant un diamètre compris entre 30 et 300 microns est supérieure à 20%, et
dans lequel l'hydrogel hétérogène hybride a un caractère viscoélastique et possède un module élastique compris entre 1 et 200kPa.

2. Hydrogel hétérogène hybride selon la revendication 1 dans lequel le réseau poreux possède une fraction des pores ayant un diamètre compris entre 30 et 300 microns qui est supérieure à 20% et une fraction des pores ayant un diamètre compris entre 10 et 30 microns qui est supérieure à 60%.

3. Hydrogel hétérogène hybride selon l'une des revendications précédentes dans lequel les microbilles comportent au moins 90% en poids du composé acrylamide.

4. Hydrogel hétérogène hybride selon la revendication 3 dans lequel les microbilles sont constituées du composé acrylamide réticulé.

5. Hydrogel hétérogène hybride selon l'une des revendications précédentes dans lequel les agrégats contiennent entre 10 et 30 microbilles.

6. Hydrogel hétérogène hybride selon l'une des revendications précédentes dans lequel les agrégats présentent une configuration en « grappe de raisin », chaque agrégat possédant une section évoluant de manière croissante d'une extrémité à l'autre selon une longueur dudit agrégat avec une section sensiblement circulaire dans un plan de coupe perpendiculaire à la longueur dudit agrégat.

7. Hydrogel hétérogène hybride selon l'une des revendications précédentes dans lequel le monomère dendrimère fonctionnalisé comporte une branche dendritique macromoléculaire de poly(oxyde d'éthylène), ladite branche dendritique macromoléculaire de poly(oxyde d'éthylène) étant fonctionnalisée par un ou plusieurs matériaux copolymérisables bioactifs choisis parmi le groupe consistant en un dérivé d'un sucre complexe, un dérivé d'un peptide d'adhérence à un tissu et un dérivé d'un conjugué polymère couplé à des anticorps dirigés contre des dérivés lipidiques lesdits un ou plusieurs matériaux copolymérisables bioactifs recouvrant la paroi du réseau percolant tridimensionnel.

8. Hydrogel hétérogène hybride selon la revendication précédente dans lequel la paroi du réseau percolant tridimensionnel est fonctionnalisée au moyen de plusieurs matériaux copolymérisables bioactifs différents.

9. Hydrogel hétérogène hybride selon l'une des revendications précédentes dans lequel lesdites une ou plusieurs molécules actives sont choisies parmi le groupe consistant en un dérivé de sucres complexes, de dérivés de peptide d'adhérence à un tissu ou de peptide avec une activité angiogénique, de dérivés de peptide de stimulation de la repousse nerveuse, de dérivés de peptide de stimulation de la prolifération et la différenciation cellulaire, un dérivé d'un conjugué polymère couplé à des anticorps dirigés contre des dérivés lipidiques, des chimiokines de la classe des Stromal-derived factor-1 (SDF-1).

10. Hydrogel hétérogène hybride selon l'une des revendications précédentes dans lequel le monomère dendrimère fonctionnalisé comporte un noyau central A, des branches dendritiques macromoléculaire de poly(oxyde d'éthylène) avec une branche dendritique fonctionnalisée par un radical acrylate ou méthacrylate polymérisable.

11. Hydrogel hétérogène hybride selon l'une des revendications précédentes dans lequel le composé acrylamide est le N-(2-hydroxypropyl)méthacrylamide (HPMA) et le monomère dendrimère fonctionnalisé comporte des branches dendritiques de poly(oxyde d'éthylène) fonctionnalisées à leur périphérie avec des agents bioactifs conférant des propriétés bioactives pour la régénération tissulaire.

12. Procédé de fabrication d'un hydrogel hétérogène hybride comportant :
- la formation de microbilles par séparation de phase induite par copolymérisation et copolymérisation radicalaire libre à une température comprise entre 45°C et 55°C à partir d'un mélange réactionnel comportant au moins trois des monomères suivants :
- un monomère dendrimère fonctionnalisé par une seule branche comportant un radical éthylénique insaturé, les autres branches étant dépourvues de radical éthylénique,
- un composé acrylamide choisi parmi un méthacrylamide N-substitué et acrylamide N-substitué, et
- au moins un agent de réticulation éthylénique insaturé bifonctionnel comportant deux liaisons vinyliques réactives, et
un initiateur à radicaux libres
les microbilles définissant des micropores fermés et non communicants dont la taille est inférieure à 20nm,
les microbilles ayant un diamètre supérieur à 1,5 microns et inférieur à 10 microns et contenant majoritairement en poids le méthacrylamide N-substitué et l'acrylamide N-substitué,
- les microbilles étant assemblées les unes aux autres pour former des agrégats contenant entre 5 et 50 microbilles, les agrégats étant liés les uns aux autres par des points de réticulation pour définir un hydrogel hétérogène hybride délimitant un réseau poreux traversant définissant des chemins percolants tridimensionnels, le réseau poreux traversant définissant des pores dont la majorité de la fraction poreuse est formée par des pores dont le diamètre est compris entre 10 et 30 microns et dont la fraction des pores ayant un diamètre compris entre 30 et 300 microns est supérieure à 20% et
dans lequel l'hydrogel hétérogène hybride a un caractère viscoélastique et possède un module élastique compris entre 1 et 200kPa ;
dans lequel la polymérisation est réalisée avec un premier plateau de température puis un deuxième plateau ou une rampe en température pour former les microbilles, la température du plateau étant comprise entre 45°C et 55°C et le deuxième plateau ou la rampe en température possédant une température supérieure d'au moins 5°C à la température du premier plateau et dans lequel le mélange réactionnel est injecté dans des moules de forme cylindrique étanche et conducteur de la chaleur.

13. Procédé de fabrication d'un hydrogel hétérogène hybride selon la revendication précédente dans lequel le rapport molaire entre le monomère dendrimère fonctionnalisé et l'agent de réticulation est compris entre 0,1 et 0,8.

14. Procédé de fabrication d'un hydrogel hétérogène hybride selon la revendication précédente dans lequel le monomère dendrimère fonctionnalisé possède une masse moléculaire est comprise entre 6220g/mol et 23280g/mol.

15. Procédé de fabrication d'un hydrogel hétérogène hybride selon l'une des revendications 12 à 14 dans lequel un matériau copolymérisable bioactif est présent dans le mélange réactionnel formant les microbilles, le matériau copolymérisable bioactif étant choisi parmi le groupe consistant en un dérivé d'un sucre complexe, un dérivé d'un peptide d'adhérence à un tissu et un dérivé d'un conjugué polymère couplé à des anticorps dirigés contre des dérivés lipidiques.

16. Procédé de fabrication d'un hydrogel hétérogène hybride selon l'une des revendications 12 à 15 dans lequel le mélange réactionnel est injecté dans un moule en métal dont les parois internes sont recouvertes de polytétrafluoroéthylène et dans lequel le moule est préférentiellement chauffé au moyen d'un bain-marie.

17. Procédé de fabrication d'un hydrogel hétérogène hybride selon l'une des revendications 12 à 16 comportant la polymérisation du mélange réactionnel à une première température pendant au moins 80 minutes pour former les microbilles puis une augmentation de la température du moule et du mélange réactionnel d'au moins 5°C.

18. Procédé de fabrication d'un implant de comblement comportant la formation d'agrégats de microbilles d'un hydrogel hétérogène hybride selon la revendication 12 et l'assemblage des agrégats les uns aux autres par un procédé d'impression tridimensionnelle pour former un implant de comblement.

## Patentansprüche

1. Hybrides heterogenes Hydrogel, das aus einem Copolymer gebildet ist, das aus mindestens drei der folgenden Monomere abgeleitet ist:
- ein Dendrimer-Monomer, das durch einen einzigen Zweig mit einem ethylenisch ungesättigten Radikal funktionalisiert ist,
- eine Acrylamidverbindung, die aus einem N-substituiertem Methacrylamid und einem N-substituiertem Acrylamid gewählt ist, und
- ein Vernetzungsmittel,
wobei das hybride heterogene Hydrogel überwiegend aus einer Vielzahl von Mikroperlen gebildet ist, die einen Durchmesser größer als 1,5 Mikrometer und keiner als 10 Mikrometer haben und gewichtsmäßig überwiegend das N-substituierte Methacrylamid und das N-substituierte Acrylamid enthalten,
wobei die Mikroperlen aneinandergefügt sind, um ein durchgehendes poröses Netz zu definieren, das dreidimensionale Perkolationspfade definiert, wobei das durchgehende poröse Netz Poren definiert, deren hauptsächlicher Porenanteil durch Poren mit einem Durchmesser zwischen 10 und 30 Mikrometer gebildet wird,
wobei das hybride heterogene Hydrogel **dadurch gekennzeichnet ist, dass** die Mikroperlen geschlossene und nicht kommunizierende Mikroporen definieren, deren Größe kleiner als 20 nm ist, die Mikroperlen aneinandergefügt sind, um Aggregate zu bilden, die zwischen 5 und 50 Mikroperlen enthalten, wobei die Aggregate eine höhere Vernetzungsdichte aufweisen als schwächer vernetzte Bereiche, die eine Bewegung der Aggregate relativ zueinander zulassen,
wobei der Porenanteil mit einem Durchmesser zwischen 30 und 300 Mikrometer größer als 20 % ist, und
wobei das hybride heterogene Hydrogel eine viskoelastische Beschaffenheit hat und ein Elastizitätsmodul zwischen 1 und 200 kPa aufweist.

2. Hybrides heterogenes Hydrogel nach Anspruch 1, wobei das poröse Netz einen Porenanteil mit einem Durchmesser zwischen 30 und 300 Mikrometer aufweist, der größer als 20 % ist, und einen Porenanteil mit einem Durchmesser zwischen 10 und 30 Mikrometer, der größer als 60 % ist.

3. Hybrides heterogenes Hydrogel nach einem der vorherigen Ansprüche, wobei die Mikroperlen mindestens 90 Gew.-% der Acrylamidverbindung umfassen.

4. Hybrides heterogenes Hydrogel nach Anspruch 3, wobei die Mikroperlen aus der vernetzten Acrylamidverbindung bestehen.

5. Hybrides heterogenes Hydrogel nach einem der vorherigen Ansprüche, wobei die Aggregate zwischen 10 und 30 Mikroperlen enthalten.

6. Hybrides heterogenes Hydrogel nach einem der vorherigen Ansprüche, wobei die Aggregate eine "Weintrauben"-Konfiguration aufweisen, wobei jedes Aggregat einen Querschnitt hat, der von einem Ende zum anderen entlang einer Länge des Aggregats mit einem im Wesentlichen kreisförmigen Querschnitt in einer Schnittebene senkrecht zur Länge des Aggregats zunehmend ist.

7. Hybrides heterogenes Hydrogel nach einem der vorherigen Ansprüche, wobei das funktionalisierte Dendrimer-Monomer einen makromolekularen dendritischen Poly(ethylenoxid)-Zweig umfasst, wobei der makromolekulare dendritische Poly(ethylenoxid)-Zweig durch ein oder mehrere bioaktive copolymerisierbare Materialien funktionalisiert ist, die aus der Gruppe gewählt sind, die aus einem Derivat eines komplexen Zuckers, einem Derivat eines Peptids zur Adhäsion an ein Gewebe und einem Derivat eines Polymerkonjugats besteht, das an Antikörper gegen Lipidderivate gekoppelt ist, wobei das eine oder die mehreren bioaktiven copolymerisierbaren Materialien die Wand des dreidimensionalen Perkolationsnetzes bedecken.

8. Hybrides heterogenes Hydrogel nach dem vorherigen Anspruch, wobei die Wand des dreidimensionalen Perkolationsnetzes durch mehrere verschiedene bioaktive copolymerisierbare Materialien funktionalisiert ist.

9. Hybrides heterogenes Hydrogel nach einem der vorherigen Ansprüche, wobei das eine oder die mehreren aktiven Moleküle aus der Gruppe gewählt sind, die aus einem Derivat komplexer Zucker, Derivaten eines Peptids zur Adhäsion an ein Gewebe oder eines Peptids mit einer angiogenen Aktivität, Derivaten eines Peptids zur Stimulierung des Nervenwachstums, Derivaten eines Peptids zur Stimulierung der Zellproliferation und -differenzierung, einem Derivat eines Polymerkonjugats, das an Antikörper gegen Lipidderivate gekoppelt ist, Chemokinen aus der Klasse der Stromzellfaktoren-1 (SDF-1) besteht.

10. Hybrides heterogenes Hydrogel nach einem der vorherigen Ansprüche, wobei das funktionalisierte Dendrimer-Monomer einen zentralen Kern A und makromolekulare dendritische Poly(ethylenoxid)-Zweige mit einem dendritischen Zweig umfasst, der durch ein polymerisierbares Acrylat- oder Methacrylatradikal funktionalisiert ist.

11. Hybrides heterogenes Hydrogel nach einem der vorherigen Ansprüche, wobei die Acrylamidverbindung N-(2-Hydroxypropyl)methacrylamid (HPMA) ist und das funktionalisierte Dendrimer-Monomer dendritische Poly(ethylenoxid)-Zweige umfasst, die an ihrem Umfang mit bioaktiven Mitteln funktionalisiert sind, die bioaktive Eigenschaften für die Geweberegeneration verleihen.

12. Verfahren zur Herstellung eines hybriden heterogenen Hydrogels, umfassend:
- Bilden von Mikroperlen durch eine Phasentrennung, die durch Copolymerisation und freie radikalische Copolymerisation bei einer Temperatur zwischen 45°C und 55°C aus einem Reaktionsgemisch induziert wird, das mindestens drei der folgenden Monomere umfasst:
- ein Dendrimer-Monomer, das durch einen einzigen Zweig funktionalisiert ist, der ein ethylenisch ungesättigtes Radikal umfasst, wobei die anderen Zweige frei von einem ethylenischen Radikal sind,
- eine Acrylamidverbindung, die aus einem N-substituiertem Methacrylamid und einem N-substituiertem Acrylamid gewählt ist, und
- mindestens ein bifunktionelles ethylenisch ungesättigtes Vernetzungsmittel, das zwei reaktive Vinylbindungen umfasst, und
einen Initiator mit freien Radikalen
wobei die Mikroperlen geschlossene und nicht kommunizierende Mikroporen definieren, deren Größe kleiner als 20 nm ist,
wobei die Mikroperlen einen Durchmesser größer als 1,5 Mikrometer und keiner als 10 Mikrometer haben und gewichtsmäßig überwiegend das N-substituierte Methacrylamid und das N-substituierte Acrylamid enthalten,
- wobei die Mikroperlen aneinandergefügt sind, um Aggregate zu bilden, die zwischen 5 und 50 Mikroperlen enthalten, wobei die Aggregate durch Vernetzungspunkte miteinander verbunden sind, um ein hybrides heterogenes Hydrogel zu definieren, das ein durchgehendes poröses Netz begrenzt, das dreidimensionale Perkolationspfade definiert, wobei das durchgehende poröse Netz Poren definiert, deren hauptsächlicher Porenanteil durch Poren mit einem Durchmesser zwischen 10 und 30 Mikrometer gebildet wird, und wobei der Porenanteil mit einem Durchmesser zwischen 30 und 300 Mikrometer größer als 20 % ist, und
wobei das hybride heterogene Hydrogel eine viskoelastische Beschaffenheit hat und ein Elastizitätsmodul zwischen 1 und 200 kPa aufweist;
wobei die Polymerisation mit einem ersten Temperaturplateau und dann einem zweiten Plateau oder einer Temperaturrampe durchgeführt wird, um die Mikroperlen zu bilden, wobei die Temperatur des Plateaus zwischen 45 °C und 55 °C liegt und das zweite Plateau oder die Temperaturrampe eine Temperatur aufweist, die mindestens 5°C höher als die Temperatur des ersten Plateaus ist, und
wobei das Reaktionsgemisch in dichte und wärmeleitende zylindrische Formen eingespritzt wird.

13. Verfahren zur Herstellung eines hybriden heterogenen Hydrogels nach dem vorherigen Anspruch, wobei das Molverhältnis zwischen dem funktionalisierten Dendrimer-Monomer und dem Vernetzungsmittel zwischen 0,1 und 0,8 liegt.

14. Verfahren zur Herstellung eines hybriden heterogenen Hydrogels nach dem vorherigen Anspruch, wobei das funktionalisierte Dendrimer-Monomer ein Molekulargewicht zwischen 6220 g/mol und 23280 g/mol aufweist.

15. Verfahren zur Herstellung eines hybriden heterogenen Hydrogels nach einem der Ansprüche 12 bis 14, wobei ein bioaktives copolymerisierbares Material im Reaktionsgemisch zur Bildung der Mikroperlen vorhanden ist, wobei das bioaktive copolymerisierbare Material aus der Gruppe gewählt ist, die aus einem Derivat eines komplexen Zuckers, einem Derivat eines Peptids zur Adhäsion an ein Gewebe und einem Derivat eines Polymerkonjugats besteht, das an Antikörper gegen Lipidderivate gekoppelt ist.

16. Verfahren zur Herstellung eines hybriden heterogenen Hydrogels nach einem der Ansprüche 12 bis 15, wobei das Reaktionsgemisch in eine Form aus Metall eingespritzt wird, deren Innenwände mit Polytetrafluorethylen bedeckt sind, und wobei die Form bevorzugt durch ein Wasserbad erhitzt wird.

17. Verfahren zur Herstellung eines hybriden heterogenen Hydrogels nach einem der Ansprüche 12 bis 16, umfassend die Polymerisation des Reaktionsgemischs bei einer ersten Temperatur mindestens 80 Minuten lang, um die Mikroperlen zu bilden, dann ein Erhöhen der Temperatur der Form und des Reaktionsgemischs um mindestens 5 °C.

18. Verfahren zur Herstellung eines Füllimplantats, umfassend das Bilden von Aggregaten aus Mikroperlen eines hybriden heterogenen Hydrogels nach Anspruch 12 und das Aneinanderfügen der Aggregate durch ein dreidimensionales Druckverfahren, um ein Füllimplantat zu bilden.

## Claims

1. Hybrid heterogeneous hydrogel formed by means of a copolymer derived from at least three of the following monomers:
- a dendrimer monomer functionalised by a single branch provided with an unsaturated ethylene radical,
- an acrylamide compound chosen from an N-substituted methacrylamide and an N-substituted acrylamide, and
- a cross-linking agent,
wherein the hybrid heterogeneous hydrogel is formed mainly by a plurality of microbeads having a diameter of more than 1.5 microns and less than 10 microns and mainly containing by weight the N-substituted methacrylamide and the N-substituted acrylamide,
wherein the microbeads are assembled to define a through porous array defining three-dimensional percolating paths,
wherein the through porous array defines pores the majority of the porous fraction whereof is formed by pores having a diameter comprised between 10 and 30 microns,
hybrid heterogeneous hydrogel **characterized in that** the microbeads define closed non-communicating micropores having a size that is smaller than 20nm, the microbeads being assembled to one another to form aggregates containing between 5 and 50 microbeads, the aggregates presenting a cross-link density greater than weakly cross-linked areas allowing shifting of the aggregates in relation to each other,
wherein the fraction of pores having a diameter comprised between 30 and 300 microns is more than 20%, and
wherein the hybrid heterogeneous hydrogel has a viscoelastic nature and has a modulus of elasticity comprised between 1 and 200kPa.

2. Hybrid heterogeneous hydrogel according to claim 1 wherein the through porous array has a fraction of pores having a diameter comprised between 30 and 300 microns that is more than 20% and a fraction of pores having a diameter comprised between 10 and 30 microns that is more than 60%.

3. Hybrid heterogeneous hydrogel according to one of the foregoing claims wherein the microbeads comprise at least 90% by weight of the acrylamide compound.

4. Hybrid heterogeneous hydrogel according to claim 3 wherein the microbeads are constituted by the cross-linked acrylamide compound.

5. Hybrid heterogeneous hydrogel according to one of the foregoing claims wherein the aggregates contain between 10 and 30 microbeads.

6. Hybrid heterogeneous hydrogel according to one of the foregoing claims wherein the aggregates present a "bunch of grapes" conformation, each aggregate has an increasing cross-section from one end to the other end along a length of said aggregate with a cross-section substantially circular in a sectional plan perpendicularly to the length of the aggregate.

7. Hybrid heterogeneous hydrogel according to one of the foregoing claims wherein the functionalised dendrimer monomer comprises one macromolecular dendritic branch of polyoxyethylene, said macromolecular dendritic branch of polyoxyethylene being functionalised by one or more bioactive copolymerisable materials chosen from the group consisting of a derivative of a complex sugar, a derivative of a tissue adhesion peptide and a derivative of a polymer conjugate coupled with antibodies directed against lipid derivatives, said one or more bioactive copolymerisable materials covering the wall of the three-dimensional percolating array.

8. Hybrid heterogeneous hydrogel according to the foregoing claim wherein the wall of the three-dimensional percolating array is functionalised by means of several different bioactive copolymerisable materials.

9. Hybrid heterogeneous hydrogel according to one of the foregoing claims wherein said one or more active molecules are chosen from the group consisting of a derivative of complex sugars, derivatives of a tissue adhesion peptide or of a peptide with an angiogenic activity, derivatives of a peptide stimulating nerve regrowth, derivatives of a peptide stimulating cell proliferation and differentiation, a derivative of a polymer conjugate coupled with antibodies directed against lipid derivatives, and chemokines of the Stromal-derived factor-1 (SDF-1) class.

10. Hybrid heterogeneous hydrogel according to one of the foregoing claims wherein the functionalised dendrimer monomer comprises a central core A, and macromolecular dendritic branches of polyoxyethylene with one dendritic branch functionalised by a polymerisable acrylate or methacrylate radical.

11. Hybrid heterogeneous hydrogel according to one of the foregoing claims wherein the acrylamide compound is N-(2-hydroxypropyl)methacrylamide (HPMA) and the functionalised dendrimer monomer comprises dendritic branches of polyoxyethylene functionalised at their periphery with bioactive agents conferring bioactive properties for tissue regeneration.

12. Method for fabricating a hybrid heterogeneous hydrogel comprising:
- forming microbeads by phase separation induced by copolymerisation and free radical copolymerisation at a temperature comprised between 45°C and 55°C from a reactional mixture comprising at least three of the following monomers:
- a dendrimer monomer functionalised by a single branch comprising an unsaturated ethylene radical, the other branches being devoid of ethylene radicals,
- an acrylamide compound chosen from an N-substituted methacrylamide and N-substituted acrylamide, and
- at least one bifunctional unsaturated ethylene cross-linking agent comprising two reactive vinyl bonds, and
a free radical initiator,
the microbeads defining closed non-communicating micropores having a size that is smaller than 20nm,
the microbeads having a diameter of more than 1.5 microns and less than 10 microns and mainly containing by weight the N-substituted methacrylamide and the N-substituted acrylamide,
the microbeads being assembled to one another to form aggregates containing between 5 and 50 microbeads, the aggregates being joined to one another by cross-linking points to define a hybrid heterogeneous hydrogel delineating a through porous array defining three-dimensional percolating paths, the through porous array defining pores the majority of the porous fraction whereof is formed by pores having a diameter comprised between 10 and 30 microns and the fraction of pores whereof having a diameter comprised between 30 and 300 microns is more than 20% and
wherein the hybrid heterogeneous hydrogel has a viscoelastic nature and has a modulus of elasticity comprised between 1 and 200kPa;
wherein polymerisation is performed with a first temperature plateau followed by a second plateau or a temperature gradient to form the microbeads, the temperature of the first plateau being comprised between 45°C and 55°C and the second plateau or the temperature gradient having a temperature at least 5°C higher than the temperature of the first plateau and
wherein the reactional mixture is injected into tight cylindrical-shaped, heat-conducting moulds.

13. Method for fabricating a hybrid heterogeneous hydrogel according to the foregoing claim wherein the molar ratio between the functionalised dendrimer monomer and the cross-linking agent is comprised between 0.1 and 0.8.

14. Method for fabricating a hybrid heterogeneous hydrogel according to the foregoing claim wherein the functionalised dendrimer monomer has a molecular mass comprised between 6,220g/mol and 23,280g/mol.

15. Method for fabricating a hybrid heterogeneous hydrogel according to one of claims 12 to 14 wherein a bioactive copolymerisable material is present in the reactional mixture forming the microbeads, the bioactive copolymerisable material being chosen from the group consisting of a derivative of a complex sugar, a derivative of a tissue adhesion peptide and a derivative of a polymer conjugate coupled with antibodies directed against lipid derivatives.

16. Method for fabricating a hybrid heterogeneous hydrogel according to one of claims 12 to 15 wherein the reactional mixture is injected into a mould made from the inner walls of which are covered by polytetrafluoroethylene and wherein the mould is preferentially heated by means of a water bath.

17. Method for fabricating a hybrid heterogeneous hydrogel according to one of claims 12 to 16 comprising polymerisation of the reactional mixture at a first temperature for at least 80 minutes to form the microbeads followed by a temperature increase of the mould and of the reactional mixture by at least 5°C.

18. Method for fabricating a filler implant comprising formation of microbead aggregates of a hybrid heterogeneous hydrogel according to claim 12 and assembly of the aggregates to one another by a three-dimensional printing method to form a filler implant.
